# EUROPEAN PATENT APPLICATION

(11) **EP 4 692 075 A1**
(43) Date of publication of application: **11.02.2026**
(21) Application number: 24193353.0
(22) Date of filing: 07.08.2024
(51) Int. Cl.: C07D 403/04

(54) **USE OF A MECHANICAL FORCE IN THE PROCESS OF PREPARATION OF OSIMERTINIB**

(71) Applicant: Université de Montpellier, 34090 Montpellier (FR); Radboud University, 6525 AJ Nijmegen (NL); Ecole Nationale Supérieure de Chimie de Montpellier, 34296 Montpellier (FR); Centre National de la Recherche Scientifique, 75016 Paris (FR)
(72) Inventor: COLACINO, Evelina, 34090 Montpellier (FR); ORT, Florian, 6711 NZ EDE (NL); BLANCO-ANIA, Daniel, 3541 DC UTRECHT (NL); RUTJES, Floris, 6605 DD WIJCHEN (NL); SOLORZANO-RODRIGUEZ, Ruben, 34170 Castelnau-le-Lez, France (FR); CHATZIGIANNIS, Christos, 34090 Montpellier (FR)
(74) Representative: Cabinet Grosset-Fournier & Demachy

(57) **Abstract**

The invention relates to the use of a mechanical force to generate a mechanochemical reaction in the implementation of a process of preparation, of N-(2-{[2(dimethylamino)ethyl](methyl)amino}-4-methoxy-5-{[4-(1-methyl-1H-indol-3-yl)pyrimidin-2-yl]amino}phenyl)prop-2-enamide (osimertinib) or a pharmaceutically acceptable salt thereof, comprising n steps, n being 5 or 6, from 1-methylindole, wherein at least one step is mechanochemical.

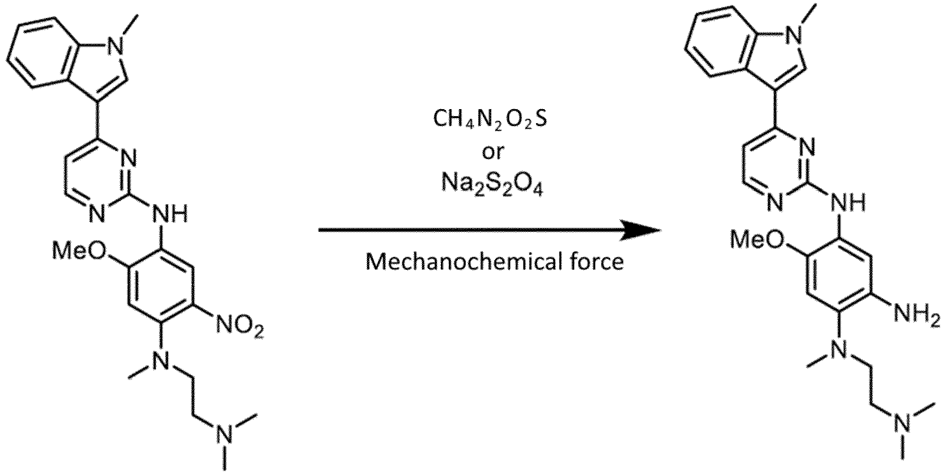

## Description

The present invention refers to the use of a mechanical force to generate a mechanochemical reaction in the implementation of a process of preparation *of N-(2-*{[2(dimethylamino)ethyl](methyl)amino}-4-methoxy-5-{[4-(1-methyl-1*H*-indol-3-yl)pyrimidin-2-yl]amino}phenyl)prop-2-enamide (osimertinib) and their process of preparation.

### Background of the Invention

Osimertinib, a third-generation Epidermal growth factor receptor (EGFR) inhibitor, is highly effective against specific mutated forms of the epidermal growth factor receptor, including L858R, Exon19 deletion, and T790M mutations. Developed by AstraZeneca, it gained accelerated Food and Drug Administrion (FDA) approval in 2015 for treating non-small cell lung carcinoma. The drug covalently binds to EGFR, targeting the T790M mutation while sparing the wild-type form. Sold under the brand name Tagrisso^{®} (Osimertinib mesylate), it is available in 40 mg and 80 mg tablets, taken daily with or without food, until disease progression or intolerable side effects occur. Tagrisso^{®} ranked among the top 25 best-selling pharmaceuticals of 2023, with nearly $5.8 billion in sales.

The current manufacturing route of Tagrisso^{®}, developed by AstraZeneca, is based on classical solution chemistry. In this route, inherent drawbacks of solution-based processes are present, such as: (1) the use of large amounts of solvents, which generate toxic waste, (2) the use of hazardous chemicals, such as acyl chlorides, organic amines as bases or metal-catalyzed processes, (3) the use of energy-intensive methods for heating/cooling reaction mixtures, or (4) the use of laborious purification methods, which often generate large amounts of toxic waste.

Over the past century, intensified human activities involving chemistry have led to significant environmental issues, including ozone layer depletion, global warming, air pollution, and overexploitation of natural resources. In response, several various measures have been implemented over the last 50 years to mitigate the negative environmental impacts of chemical production.

Acknowledging the necessity to lessen the environmental damage caused by the chemical industry has spurred the growth of mechanochemistry.

This approach seeks to safeguard future generations by advancing more sustainable chemical processes. Mechanochemistry allows reactions to occur without solvents or with minimal liquid media, in a process named Liquid Assisted Grinding (LAG) making it a viable alternative for green chemical synthesis. Its advantages include the absence of bulk solvents, precise stoichiometric control (using reagents in exact amounts rather than excess), and more selective reactions, which simplify work-up procedures, thus reducing the amount of total waste generated. These features highlight the superiority of mechanochemical processes compared to traditional solution-based chemistry.

One of the issues to address is to find new mechanochemical reactions intended to be used in the process of preparation of active pharmaceutical ingredient.

One of the aims of the invention is to provide new use of a mechanical force to conduct a mechanochemical total synthesis to provide osimertinib or a pharmaceutically acceptable salt thereof.

Another aim of the invention is to provide a mechanochemical process of preparation of osimertinib.

Another aim of the invention is to provide a mechanochemical process of salification of osimertinib by using methanesulfonic acid.

Additional aims of the invention, in comparison with the manufacturing method in solution concern:
a) shorter reaction times for each step by using BM and/or RAM
b) reduced waste for each step by using BM and/or RAM
c) reduced energetic costs (in principle) for both synthesis and/or work-up by BM and/or by RAM, by providing milder operational reaction conditions at room temperature, which avoids the use of heating/cooling procedures (used in solution) for both synthesis and/or work-up.
d) Reduce the amount of raw materials needed for the syntheses (for each step by BM and/or by RAM) using no excess of chemicals (and also no-solvents), which turns in a more economic process in terms of raw materials use. This also translate in
e) Less waste generated by BM/RAM for each step (and when they are generated, they remain less harmful as well compared to those generated by the manufacturing route in solution)
f) Simplified work-up procedures (operationally simple - no chromatography needed) and generating less waste

### Brief summary of the invention

The Applicant has surprisingly found that a mechanical force to generate a mechanochemical reaction in the implementation of a process of preparation of osimertinib can be used. Inventors were able to carry out a mechanochemical total synthesis of osimertinib mesylate in 6 steps, by implementing mechanochemical processes for its preparation in 6 steps, which is 1 step shorter than the solution-based manufacturing method that is being used in the industry.

### Detailed description of the invention

More particularly, one of the objects of the present invention is the use of a mechanical force to generate a mechanochemical reaction in the implementation of a process of preparation, of *N-*(2-{[2(dimethylamino)ethyl](methyl)amino}-4-methoxy-5-{[4-(1-methyl-1H-indol-3-yl)pyrimidin-2-yl]amino}phenyl)prop-2-enamide (osimertinib) or a pharmaceutically acceptable salt thereof, comprising n steps, n being 5 or 6, from 1-methylindole,
wherein at least one step is mechanochemical.

As used herein the expression "mechanochemical force" refers to mechanochemical activation or mechanochemistry, that is a technology and a research field in which solid chemical reactants, i.e in the form of granules or powders (pulverulent) or in the pasty state, are milled or ground together or ground together under mechanical activation and chemical reaction are induced by the direct absorption of mechanical energy, (McNaught, A.D.; Wilkinson, A. IUPAC Compendium of Chemical Terminology ("The Golden Book"); 2nd edition; Blackwell Scientific publications, Oxford, 1997) without solvents (neat grinding) or with minimum/small amounts of solvent in a process named liquid-assisted grinding (LAG) in comparison with solution-based methods. The mechanochemical reaction allows to perform chemical reactions without solvents or with minimum/small amounts of solvent. The mechanochemical reaction occurs because of the mechanochemical activation by the mechanochemical force.

*N*-(2-{[2(dimethylamino)ethyl](methyl)amino}-4-methoxy-5-{[4-(1-methyl-1H-indol-3-yl)pyrimidin-2-yl]amino}phenyl)prop-2-enamide (osimertinib, CAS number : 1421373-65-0) has the following structure :

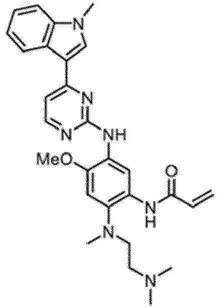

The expression "pharmaceutically acceptable salt" means all pharmaceutically or physiologically acceptable salt forms of the compounds of osimertinib which may be formed, by protonation of a nitrogen an amino function, with an inorganic or organic acid. Examples acid addition salts comprise, mineral acid salts such as hydrochloride, hydrobromide, hydroiodide, sulfate salts (such as, e.g., sulfate or hydrogensulfate salts), phosphate salts (such as hexafluorophosphate, hydrogenphosphate, or dihydrogenphosphate salts), carbonate salts, hydrogenocarbonate salts, borate salts, organic acid salts such as oxalate, fumarate, tartrate, malate, citrate, succinate, adipate, gluconate, glycolate, nicotinate, benzoate, salicylate, ascorbate salts; sulfonate salts such as methanesulfonate (mesylate), ethanesulfonate (esylate), 2-hydroxyethanesulfonate (isethionate), p-toluenesulfonate (tosylate), or camphorsulfonate salts; glycerophosphate salts; and acidic amino acid salts such as aspartate or glutamate salts. Preferred pharmaceutically/physiologically acceptable salt of osimertinib include methanesulfonate (mesylate).
"wherein at least one step is mechanochemical" refers to the fact that in the process of preparation of osimertinib, the minimum number of step that is performed thanks to mechanical activation is 1.

The starting product in the preparation of osimertinib is 1-methylindole (CAS number: 603-76-9) is an alkylated bicyclic aromatic compound containing a nitrogen. It has a bicyclic structure, consisting of a six-membered benzene ring fused to a five-membered pyrrole ring, in which the nitrogen is alkylated by a methyl group.

According to a particular embodiment of the use of the invention, the number of steps n is 5.

According to a particular embodiment of the use of the invention, the process of preparation comprises an acylation step, two subsequent nucleophilic aromatic substitution steps, a reduction step and an amidation step.

According to a particular embodiment of the use of the invention, the number of steps n is 6.

According to a particular embodiment of the use of the invention, the process of preparation comprises an acylation step, two subsequent nucleophilic aromatic substitution steps, a reduction step, an amidation step and a salification step.

According to another embodiment, the use of the invention consists in the use of a mechanical force to generate a mechanochemical reaction in the implementation of a process of preparation, of *N*-(2-{[2(dimethylamino)ethyl](methyl)amino}-4-methoxy-5-{ [4-(1-methyl-1H-indol-3-yl)pyrimidin-2-yl]amino}phenyl)prop-2-enamide (osimertinib) or a pharmaceutically acceptable salt thereof, comprising n steps, n being 6 or 7, from indole,
wherein at least one step is mechanochemical.

According to a particular embodiment of the use of the invention, the process of preparation comprises an alkylation step, an acylation step, two subsequent nucleophilic aromatic substitution steps, a reduction step and an amidation step.

According to a particular embodiment of the use of the invention, the mechanical forces are generated by a method chosen among: ball-milling (BM) or resonant acoustic mixing (RAM).

Ball-milling comprises the following devices in a non-limiting manner: vibrating ball-mill, planetary ball-mill, drum mill, dyno-mill, or vibrating eccentric mill.

Examples of the reaction vessel and the balls as an agitation medium used in the milling devices include ones formed of such materials as stainless steel, agate, alumina, tungsten carbide, chrome steel, zirconia oxide, silicon nitride and the like. Among these materials, zirconia oxide, is preferred. The size of the vessel used in the planetary ball mill is not particularly limited and may be approximately 1 cm³ to 1000 L. The size of the balls is also not particularly limited and may be approximately from 2 to 50 mm in diameter. Particularly preferred specific examples of the vibrating and/or planetary ball mill include stainless steel and zirconia oxide as reaction vessel material.

Resonant acoustic mixing (RAM) involves applying a vibration to a container to induce reciprocating up and down motions on the contents of the container. The vibration may be applied with a relatively high (up to 100 g) acceleration and a relatively low amplitude. The frequency of the vibration is typically around 60 Hz. The container can be cylindrical shaped with a circumferential side wall and disc-shaped top and bottom walls. The force is applied along the longitudinal axis of the container.

In resonant acoustic mixing, acoustic energy is delivered to the components to be mixed. An oscillating mechanical driver creates motion in a mechanical system comprising engineered plates, eccentric weights and springs. This energy is then acoustically transferred to the material to be mixed. The underlying technology principle is that the system operates at resonance. In this mode, there is a nearly complete exchange of energy between the mass elements and the elements in the mechanical system.

In a resonant acoustic mixing, the only element that absorbs energy (apart from some negligible friction losses) is the reactant composition. Thus, the resonant acoustic mixing provides a highly efficient way of transferring mechanical energy directly into the starting materials. The resonant frequency can be from about 15 Hertz to about 2000 Hertz, or from about 20 Hertz to about 1800 Hertz, or from about 20 Hertz to about 1700 Hertz. The g force applied by the acoustic mixer to the reactant composition can be from about 2 g force to about 100 g force.

Resonant acoustic mixers are available from Resodyn^{™} Acoustic Mixers and can be chosen from LabRAM I and LabRAM II and like.

According to a particular embodiment of the use of the invention at least two steps, three steps, four steps, five steps or all the steps are mechanochemical.

The expression "all the steps" means, as a matter of example and in a non-limiting manner, that six steps are mechanochemical.

According to a particular embodiment of the use of the invention, at least two steps are mechanochemical.

According to a particular embodiment of the use of the invention, the at least two steps that are mechanochemical are chosen among the following one : the acylation step and one of the nucleophilic aromatic substitution step, the acylation step and the reduction step, the acylation step and the amidation step, the acylation step and the salification step, the two subsequent nucleophilic aromatic substitution steps, a nucleophilic aromatic substitution step and the reduction step, one of the nucleophilic aromatic substitution step and the amidation step, one of the nucleophilic aromatic substitution step and the salification step, the reduction step and the amidation step, the reduction step and the salification step and the amidation step and the salification step.

According to a particular embodiment of the use of the invention, wherein at least three steps are mechanochemical.

According to a particular embodiment of the use of the invention, the at least three steps that are mechanochemical are chosen among the following one:
- the acylation step and the two subsequent nucleophilic aromatic substitution steps,
- the acylation step, one of the nucleophilic aromatic substitution steps and the reduction step,
- the acylation step, one of the nucleophilic aromatic substitution steps and the amidation step,
- the acylation step, one of the nucleophilic aromatic substitution steps and the salification step,
- the acylation step, the reduction step and the amidation step,
- the acylation step, the reduction step and the salification step,
- the acylation step, the amidation step and the salification step,
- the two subsequent nucleophilic aromatic substitution steps and the reduction step,
- the two subsequent nucleophilic aromatic substitution steps and the amidation step,
- the two subsequent nucleophilic aromatic substitution steps and the salification step,
- one of the nucleophilic aromatic substitution steps, the reduction step and the amidation step,
- one of the nucleophilic aromatic substitution steps, the reduction step and the salification step,
- one of the nucleophilic aromatic substitution steps, the amidation step and the salification step,
- the reduction step, the amidation step and the salification step.

According to a particular embodiment of the use of the invention, wherein at least four steps are mechanochemical.

According to a particular embodiment of the use of the invention, the at least four steps that are mechanochemical are chosen among the following one:
- the acylation step, one of the nucleophilic aromatic substitution steps, the nucleophilic aromatic substitution step and the reduction step,
- the acylation step, the nucleophilic aromatic substitution step, one of the nucleophilic aromatic substitution steps and the amidation step,
- the acylation step, the two subsequent nucleophilic aromatic substitution steps and the salification step,
- the acylation step, one of the nucleophilic aromatic substitution steps, the reduction step and the amidation step,
- the acylation step, one of the nucleophilic aromatic substitution steps, the reduction step and the salification step,
- the acylation step, one of the nucleophilic aromatic substitution steps, the amidation step and the salification step,
- the acylation step, the reduction step, the amidation step and the salification step,
- the two subsequent nucleophilic aromatic substitution steps, the reduction step and the amidation step,
- the two subsequent nucleophilic aromatic substitution steps, the reduction step and the salification step,
- the two subsequent nucleophilic aromatic substitution steps, the amidation step and the salification step,
- the nucleophilic aromatic substitution step, the reduction step, the amidation step and the salification step.

According to a particular embodiment of the use of the invention, wherein at least five steps are mechanochemical.

According to a particular embodiment of the use of the invention, the at least five steps that are mechanochemical are chosen among the following one:
- the acylation step, the two subsequent nucleophilic aromatic substitution steps, the reduction step and the amidation step,
- the acylation step, the two subsequent nucleophilic aromatic substitution steps, the reduction step and the salification step,
- the acylation step, the two subsequent nucleophilic aromatic substitution steps, the amidation step and the salification step,
- the acylation step, one of the nucleophilic aromatic substitution steps, the reduction step, the amidation step and the salification step,
- the two subsequent nucleophilic aromatic substitution steps, the reduction step, the amidation step and the salification step

According to a particular embodiment of the use of the invention, wherein at least six steps are mechanochemical.

According to a particular embodiment of the use of the invention, wherein an acylation step, two subsequent nucleophilic aromatic substitution steps, a reduction step, an amidation step and a salification step are mechanochemical.

According to a particular embodiment of the use of the invention, said process of preparation can provide from 0.1 g to 1 kg of osimertinib or of a pharmaceutically acceptable salt thereof. As used herein the expression "scalable" means the ability to be changed in size or scale to industrial equipment.

According to a particular embodiment of the use of the invention, said process of preparation can provide from 0.1 g to 500 g of osimertinib or of a pharmaceutically acceptable salt thereof. According to a particular embodiment of the use of the invention, said process of preparation can provide from 500 g to 1 kg of osimertinib or of a pharmaceutically acceptable salt thereof. According to a particular embodiment of the use of the invention, said osimertinib or of a pharmaceutically acceptable salt thereof being produced in an amount greater than 10 g.

According to a preferred embodiment of the use of the invention, comprising a reduction step of N'-(2-dimethylaminoethyl)-2-methoxy-N'-methyl-N-[4-(1-methylindol-3-yl)pyrimidin-2-yl]-5-nitrobenzene-1,4-diamine into N1-(2-dimethylaminoethyl)-5-methoxy-N1-methyl-N4-[4-(1-methylindol-3-yl)pyrimidin-2-yl]benzene-1,2,4-triamine, said reduction step is mechanochemical,
and/or
comprising an amidation step of the above mentioned N1-(2-dimethylaminoethyl)-5-methoxy-N1-methyl-N4-[4-(1-methylindol-3-yl)pyrimidin-2-yl]benzene-1,2,4-triamine into osimertinib, said amidation step is mechanochemical.

The expression "N'-(2-dimethylaminoethyl)-2-methoxy-N'-methyl-N-[4-(1-methylindol-3-yl)pyrimidin-2-yl]-5-nitrobenzene-1,4-diamine" refers to the following compound :

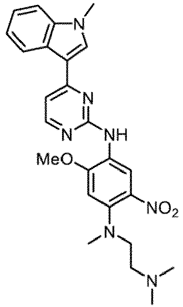

The expression "N1-(2-dimethylaminoethyl)-5-methoxy-N1-methyl-N4-[4-(1-methylindol-3-yl)pyrimidin-2-yl]benzene-1,2,4-triamine" refers to the following compound :

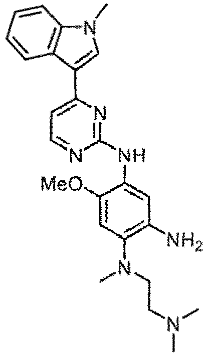

The reduction step is this embodiment consists in a reduction of the nitro group into an amine. Additional advantages of the invention are :
a) the reduction reaction in solution occurs in the presence of metals and generate halogenated (chlorinated) waste (reduction system : Fe/NH4Cl), while the reduction method by BM or RAM occurs **without metals and without halogens** (and it does not generate halogenated/chlorinated waste).
b) The reaction time for the reduction reaction in solution for the commercial route is not specified. The reaction time in solution is 2h. The reaction by BM occurs in 3h, while the reaction by RAM occurs in 30 min.

The amidation step in this embodiment consists in the introduction of a 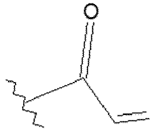 group, linked through an amide bond to the NH₂ function of the phenyle ring. The amidation step is referred to the introduction of an acrylamide group directly, which is not the way osimertinib is prepared is solution in the prior art, being the acrylamide group generated only at the end of the process of preparation of osimertinib by an elimination reaction. Indeed, acrylamide is sensitive to nucleophiles, which makes its handling difficult in the conventional synthetic steps in solution. Herein, it is introduced directly, which make the synthesis by mechanochemistry 1 step shorter.

An additional advantage of the invention concerns the amidation reaction in solution that occurs in 90 minutes. The reaction by BM last for the same time, however, by RAM, the amidation method is 60 min (thus shorter than the method in solution).

According to a particular embodiment of the use of the invention, comprising a reduction step of N'-(2-dimethylaminoethyl)-2-methoxy-N'-methyl-N-[4-(1-methylindol-3-yl)pyrimidin-2-yl]-5-nitrobenzene-1,4-diamine into N1-(2-dimethylaminoethyl)-5-methoxy-N1-methyl-N4-[4-(1-methylindol-3-yl)pyrimidin-2-yl]benzene-1,2,4-triamine, said reduction step is mechanochemical.

According to a particular embodiment of the use of the invention, comprising an amidation step of the above mentioned N1-(2-dimethylaminoethyl)-5-methoxy-N1-methyl-N4-[4-(1-methylindol-3-yl)pyrimidin-2-yl]benzene-1,2,4-triamine into osimertinib, said amidation step is mechanochemical.

Another object of the present invention is a mechanochemical process of preparation of osimertinib.

A mechanochemical process of preparation of osimertinib, which comprises the step of:
d) a mechanochemical reduction step of N'-(2-dimethylaminoethyl)-2-methoxy-N'-methyl-N-[4-(1-methylindol-3-yl)pyrimidin-2-yl]-5-nitrobenzene-1,4-diamine with sodium dithionite or thiourea dioxide, under neat grinding or in liquid-assisted grinding (LAG) conditions, using BM or RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine.

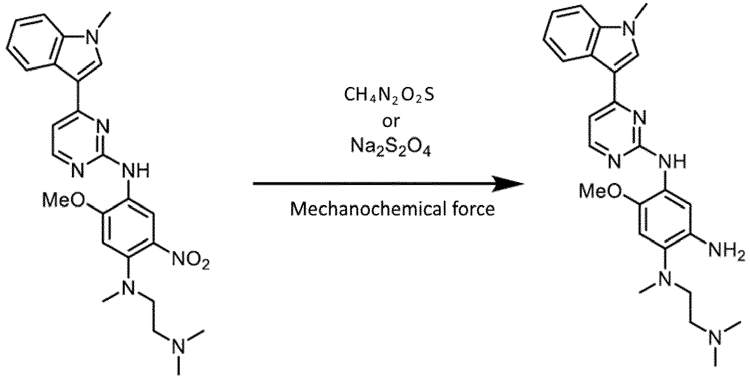

The expression "neat grinding" corresponds to the mechanical force that takes place in absence of solvent.

As used herein, the term "liquid-assisted grinding (LAG)" refers to minimum or limited amounts of a liquid, the role of which is to increase ease of mixing and/or to participate to the reaction occurring in a mechanochemical system. Under conventionally defined LAG conditions; the liquid solvent has also been described as functioning essentially as a catalyst or lubrificant.

LAG uses a small amount of a liquid to accelerate reactions, as well as to enable transformations that do not take place by neat grinding. The empirical definition of LAG is based on the way mechanochemical reactivity is affected by the ratio of the liquid solvent to the weight of chemical reactants (η, eta).

A value of η = 0 µL/mg corresponds to neat grinding, η > 2 µL/mg corresponds to a typical solution reaction, while LAG lies in the range of ≈0-1 µL/mg. In that range, reactivity appears independent of reactant solubility, distinguishing LAG from slurry reactions (1 µL/mg < η < 2 µL/mg) in which low solubility does hinder reactivity. (Friščić et al., *ACS Cent. Sci.* **2017,** 3(1), 13-19)

As used herein, the terms "minimum or limited amounts of a liquid" or "minimum /small amount of solvent" refers to an eta value (*η*) up to 1, compared to the same reaction without mechanochemical conditions".

"a crude product containing" means that during one of the step a), b), c), d), e) or f), a crude product containing various traces of unwanted reagents is obtained, and this crude product must go through a purification to give the desired product.

As a matter of example and in a non-limiting manner, the step d) can be carried out at 25°C, with a mixture of water and ethanol under LAG conditions.

According to a particular embodiment, the process of the invention comprises the steps of: d) a mechanochemical reduction step of N'-(2-dimethylaminoethyl)-2-methoxy-N'-methyl-N-[4-(1-methylindol-3-yl)pyrimidin-2-yl]-5-nitrobenzene-1,4-diamine with sodium dithionite or thiourea dioxide, under neat grinding or in liquid-assisted grinding (LAG) conditions, using BM or RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 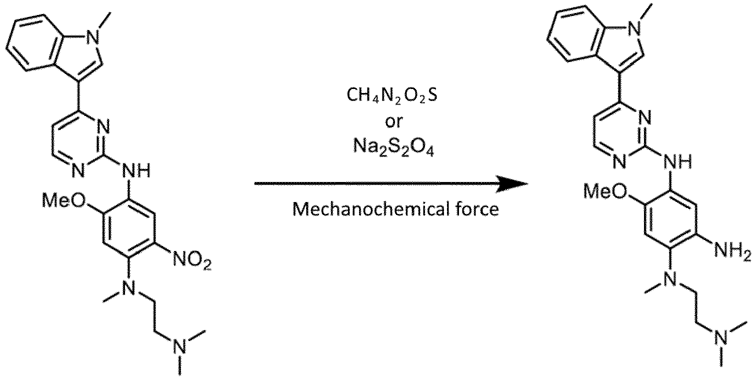 and,
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with acrylic acid, chloro-N,N,N',N'-tetramethylformamidinium hexafluorophosphate (TCFH), and tripotassium phosphate or potassium hydrogenophosphate, in liquid-assisted grinding (LAG) conditions, using BM to obtain a crude product containing osimertinib, followed by a purification of said crude product, to obtain osimertinib,
   or
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with potassium acrylate and (3-dimethylamino-propyl)-ethyl-carbodiimide hydrochloride, in liquid-assisted grinding (LAG) conditions, using RAM to obtain a crude product containing osimertinib, followed by a purification of said crude product, to obtain osimertinib,

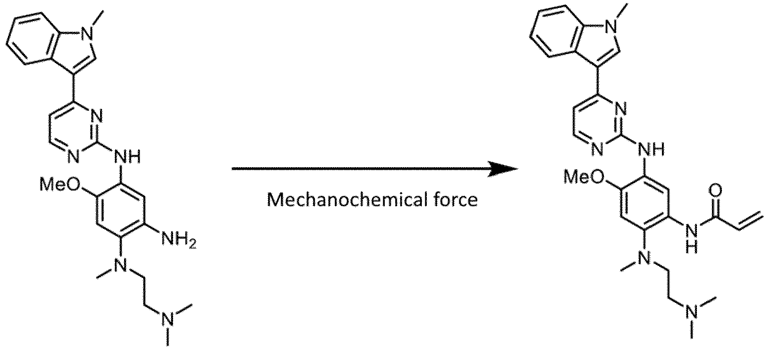

As a matter of example and in a non-limiting manner, the step e) can be carried out at 25°C, with ethyl acetate under LAG conditions.

According to a particular embodiment, the process of the invention comprises the steps of:
d) a mechanochemical reduction step of N'-(2-dimethylaminoethyl)-2-methoxy-N'-methyl-N-[4-(1-methylindol-3-yl)pyrimidin-2-yl]-5-nitrobenzene-1,4-diamine with sodium dithionite or thiourea dioxide, under neat grinding or in liquid-assisted grinding (LAG) conditions, using BM, to obtain a crude product containing N1-(2-dimethylaminoethyl)-5-methoxy-N1-methyl-N4-[4-(1-methylindol-3 -yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 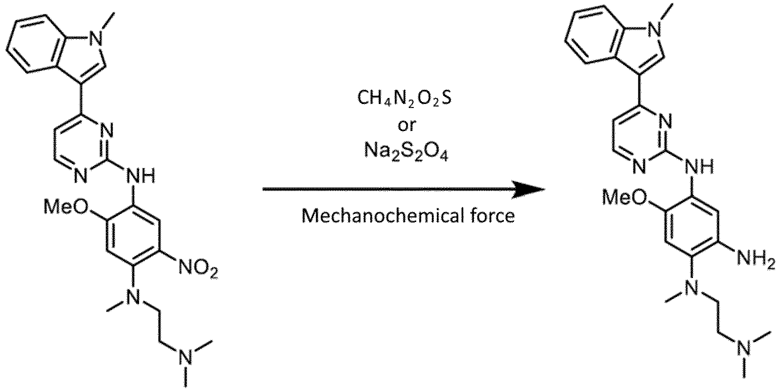 and,
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with acrylic acid, chloro-N,N,N',N'-tetramethylformamidinium hexafluorophosphate (TCFH), and tripotassium phosphate or potassium hydrogenophosphate, in liquid-assisted grinding (LAG) conditions, using BM to obtain a crude product containing osimertinib, followed by a purification of said crude product, to obtain osimertinib

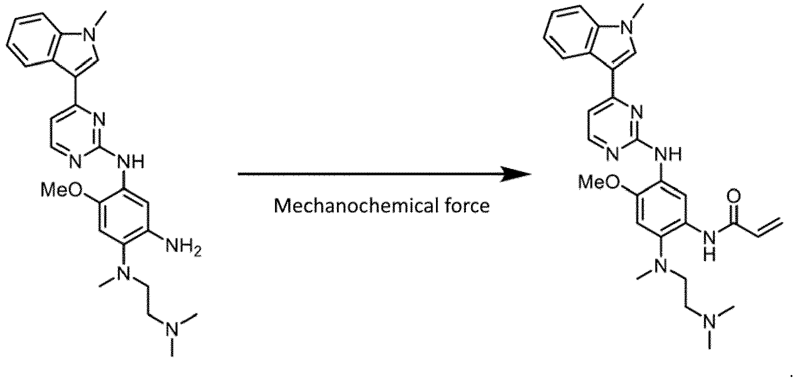

According to a particular embodiment, the process of the invention comprises the steps of:
d) a mechanochemical reduction step of N'-(2-dimethylaminoethyl)-2-methoxy-N'-methyl-N-[4-(1-methylindol-3-yl)pyrimidin-2-yl]-5-nitrobenzene-1,4-diamine with sodium dithionite or thiourea dioxide, under neat grinding or in liquid-assisted grinding (LAG) conditions, using RAM, to obtain a crude product containing N1-(2-dimethylaminoethyl)-5-methoxy-N1-methyl-N4-[4-(1-methylindol-3 -yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 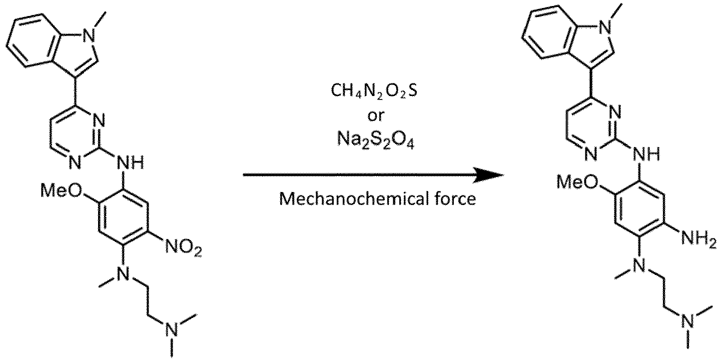 and,
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with potassium acrylate and (3-dimethylamino-propyl)-ethyl-carbodiimide hydrochloride, in liquid-assisted grinding (LAG) conditions, using RAM to obtain a crude product containing osimertinib, followed by a purification of said crude product, to obtain osimertinib

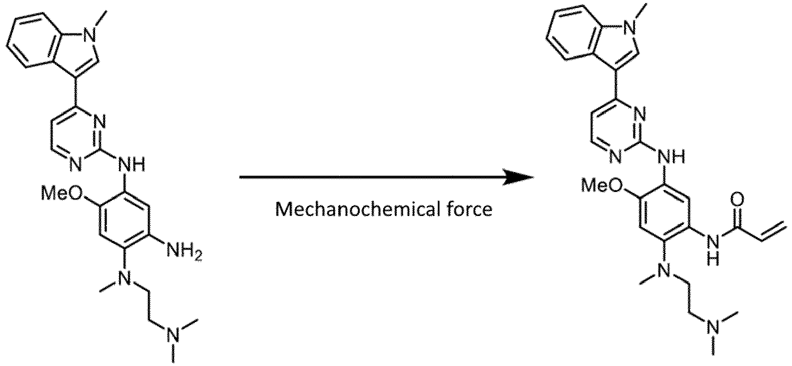

According to a preferred embodiment, the process of the invention comprises the steps of:
a) a mechanochemical Friedel-Craft acylation step of 1-methylindole with 2,4-dichloropyrimidine and a Lewis acid, using BM or RAM under neat grinding or in liquid-assisted grinding (LAG) conditions, to obtain a crude product containing 3-(2-chloropyrimidin-4-yl)-1-methyl-1H-indole, followed by a purification of said crude product, to obtain -(2-chloropyrimidin-4-yl)-1-methyl-1H-indole, 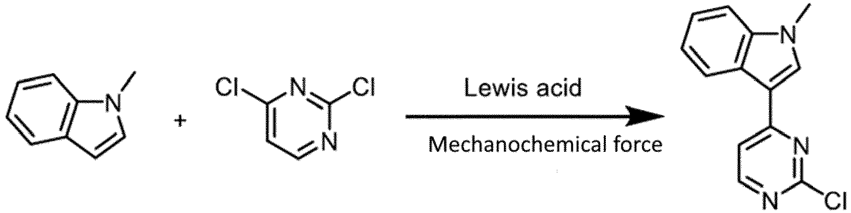
b) a mechanochemical nucleophilic aromatic substitution step of the above mentioned 3-(2-chloropyrimidin-4-yl)-1-methyl-1H-indole with 4-fluoro-2-methoxy-5-nitroaniline and a sulfonic acid, under neat grinding or in liquid-assisted grinding (LAG) conditions, using BM or RAM to obtain a crude product containing N-(4-fluoro-2-methoxy-5-nitrophenyl)-4-(1-methyl-1H-indol-3-yl)pyrimidin-2-amine, followed by a purification of said crude product, to obtain N-(4-fluoro-2-methoxy-5-nitrophenyl)-4-(1-methyl-1H-indol-3-yl)pyrimidin-2-amine, 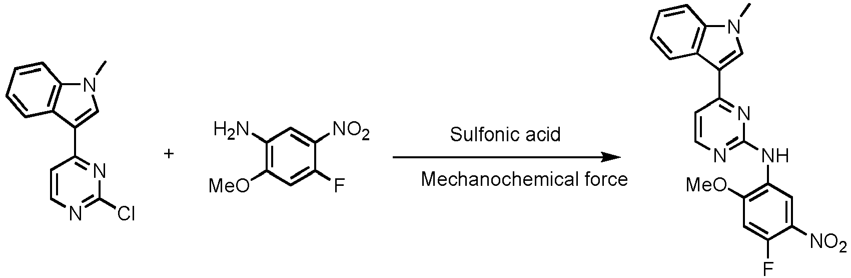
c) a mechanochemical nucleophilic aromatic substitution step of the above mentioned N-(4-fluoro-2-methoxy-5-nitrophenyl)-4-(1-methyl-1H-indol-3-yl)pyrimidin-2-amine with N,N,N'-trimethylethylenediamine and a base, in particular K₂CO₃, in liquid-assisted grinding (LAG) conditions, using BM or RAM, to obtain a crude product containing N'-(2-dimethylaminoethyl)-2-methoxy-N'-methyl-N-[4-(1-methylindol-3-yl)pyrimidin-2-yl]-5-nitrobenzene-1,4-diamine, followed by a purification of said crude product, to obtain N'-(2-dimethylaminoethyl)-2-methoxy-N'-methyl-N-[4-(1-methylindol-3-yl)pyrimidin-2-yl]-5-nitrobenzene-1,4-diamine,

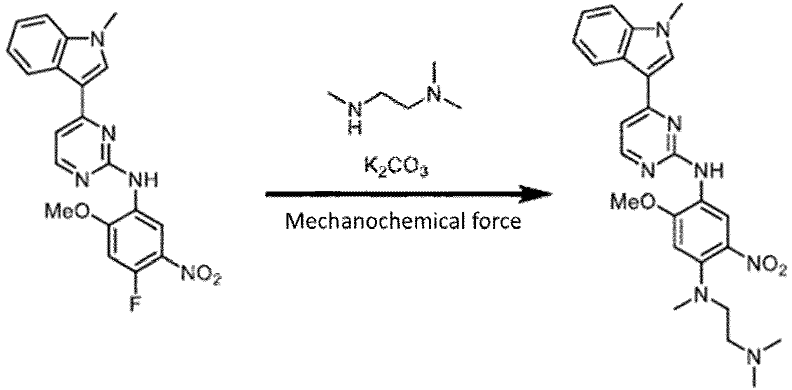

It can be noted that the reaction by BM and/or by RAM occurs at room temperature, while the reaction in solution (manufacturing route) occurs at 85°C. The present procedure is milder (no heating) than the manufacturing one in solution.
d) a mechanochemical reduction step of the above mentioned N'-(2-dimethylaminoethyl)-2-methoxy-N'-methyl-N-[4-(1-methylindol-3 -yl)pyrimidin-2-yl]-5-nitrobenzene-1,4-diamine with sodium dithionite or thiourea dioxide, under neat grinding or liquid-assisted grinding (LAG) conditions, using BM or RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 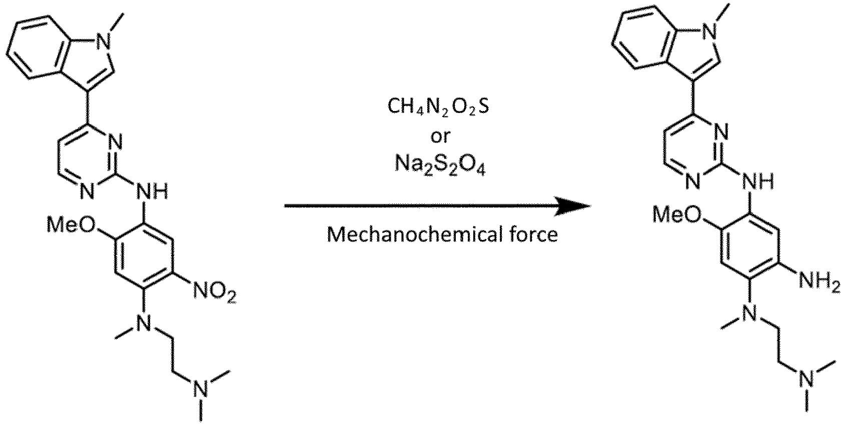 and,
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with acrylic acid, chloro-N,N,N',N'-tetramethylformamidinium hexafluorophosphate (TCFH), and tripotassium phosphate or potassium hydrogenophosphate, in liquid-assisted grinding (LAG) conditions, using BM to obtain a crude product containing osimertinib, followed by a purification of said crude product, to obtain osimertinib,
   or
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with potassium acrylate and (3-dimethylamino-propyl)-ethyl-carbodiimide hydrochloride, in liquid-assisted grinding (LAG) conditions, using RAM to obtain a crude product containing osimertinib, followed by a purification of said crude product, to obtain osimertinib,

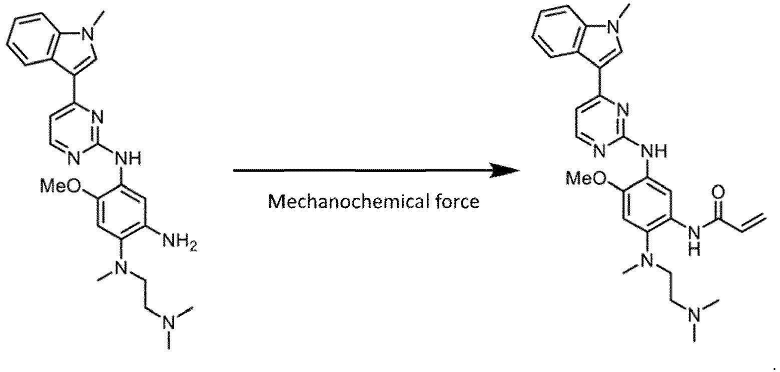

Said 2,4-dichloropyrimidine (CAS Number : 3934-20-1) involved in the step a) has the following structure :

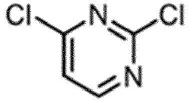

As a matter of example and in a non-limiting manner, the step a) can be carried out at 25°C under neat grinding.

Said 3-(2-chloropyrimidin-4-yl)-1-methyl-1H-indole (CAS Number: 1032452-86-0) involved in step b) has the following structure:

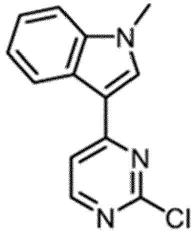

Said 4-fluoro-2-methoxy-5-nitroaniline (CAS Number: 1075705-01-9) involved in step b) has the following structure:

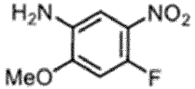

As a matter of example and in a non-limiting manner, the step b) can be carried out at 25°C under neat grinding or in LAG conditions.

Said N-(4-fluoro-2-methoxy-5-nitrophenyl)-4-(1-methyl-1H-indol-3-yl)pyrimidin-2-amine (CAS Number: 1421372-94-2) involved in step c) has the following structure:

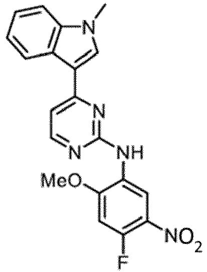

During the mechanochemical nucleophilic aromatic substitution step with 4-fluoro-2-methoxy-5-nitroaniline, a nitrogen-carbon bound is created between the NH₂ function of the phenyl ring of N-(4-fluoro-2-methoxy-5-nitrophenyl)-4-(1-methyl-1H-indol-3-yl)pyrimidin-2-amine and the carbon bearing the fluorine of 4-fluoro-2-methoxy-5-nitroaniline.

Said N,N,N'-trimethylethylenediamine (CAS number : 142-25-6) involved in step c) has the following structure :

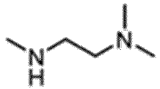

During the mechanochemical nucleophilic aromatic substitution step with N,N,N'-trimethylethylenediamine, a nitrogen-carbon bound is created between the amino function of N,N,N'-trimethylethylenediamine bearing a hydrogen and the carbon bearing the fluorine of N-(4-fluoro-2-methoxy-5-nitrophenyl)-4-(1-methyl-1H-indol-3 -yl)pyrimidin-2-amine.

As a matter of example and in a non-limiting manner, the step c) can be carried out at 25°C with dimethylsulfoxide under LAG conditions.

According to a particular embodiment, the process of the invention comprises the steps of:
a) a mechanochemical Friedel-Craft acylation step of 1-methylindole with 2,4-dichloropyrimidine and a Lewis acid, using BM under neat grinding or in liquid-assisted grinding (LAG) conditions, to obtain a crude product containing 3-(2-chloropyrimidin-4-yl)-1-methyl-1H-indole, followed by a purification of said crude product, to obtain - (2-chloropyrimidin-4-yl)-1-methyl-1H-indole, 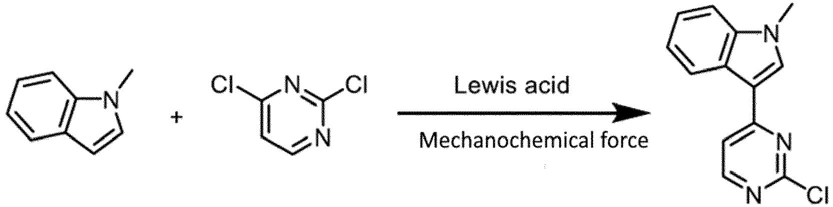
b) a mechanochemical nucleophilic aromatic substitution step of the above mentioned 3-(2-chloropyrimidin-4-yl)-1-methyl-1H-indole with 4-fluoro-2-methoxy-5-nitroaniline and a sulfonic acid, under neat grinding or in liquid-assisted grinding (LAG) conditions, using BM to obtain a crude product containing N-(4-fluoro-2-methoxy-5-nitrophenyl)-4-(1-methyl-1H-indol-3-yl)pyrimidin-2-amine, followed by a purification of said crude product, to obtain N-(4-fluoro-2-methoxy-5-nitrophenyl)-4-(1-methyl-1H-indol-3-yl)pyrimidin-2-amine, 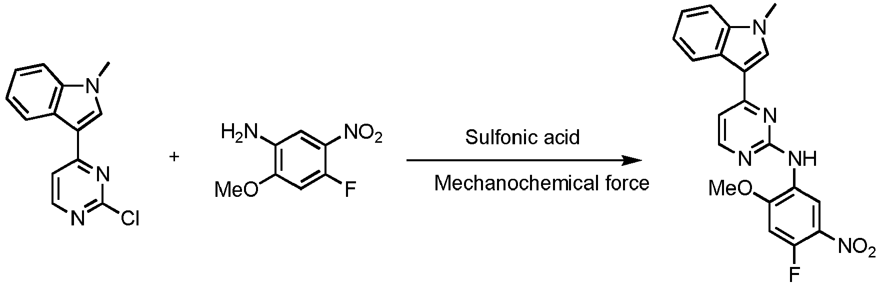
c) a mechanochemical nucleophilic aromatic substitution step of the above mentioned N-(4-fluoro-2-methoxy-5-nitrophenyl)-4-(1-methyl-1H-indol-3-yl)pyrimidin-2-amine with N,N,N'-trimethylethylenediamine and a base, in particular K₂CO₃, in liquid-assisted grinding (LAG) conditions, using BM, to obtain a crude product containing N'-(2-dimethylaminoethyl)-2-methoxy-N'-methyl-N-[4-(1-methylindol-3-yl)pyrimidin-2-yl]-5-nitrobenzene-1,4-diamine, followed by a purification of said crude product, to obtain N'-(2-dimethylaminoethyl)-2-methoxy-N'-methyl-N-[4-(1-methylindol-3-yl)pyrimidin-2-yl]-5-nitrobenzene-1,4-diamine,

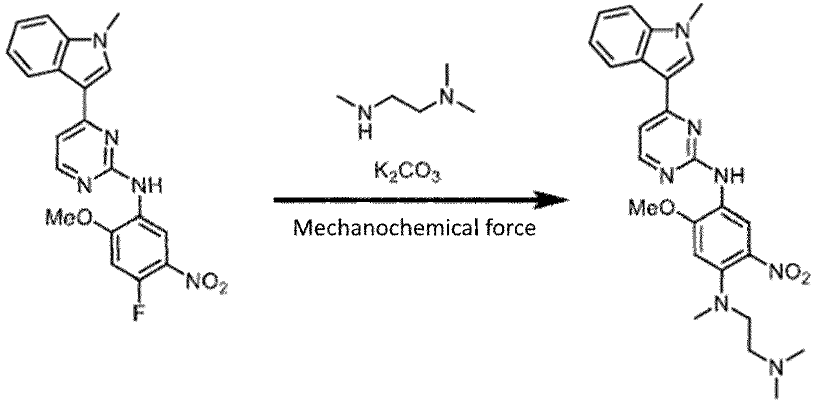

It can be noted that the reaction by BM and/or by RAM occurs at room temperature, while the reaction in solution (manufacturing route) occurs at 85°C. The present procedure is milder (no heating) than the manufacturing one in solution
d) a mechanochemical reduction step of the above mentioned N'-(2-dimethylaminoethyl)-2-methoxy-N'-methyl-N-[4-(1-methylindol-3 -yl)pyrimidin-2-yl]-5-nitrobenzene-1,4-diamine with sodium dithionite or thiourea dioxide, under neat grinding or in liquid-assisted grinding (LAG) conditions, using BM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 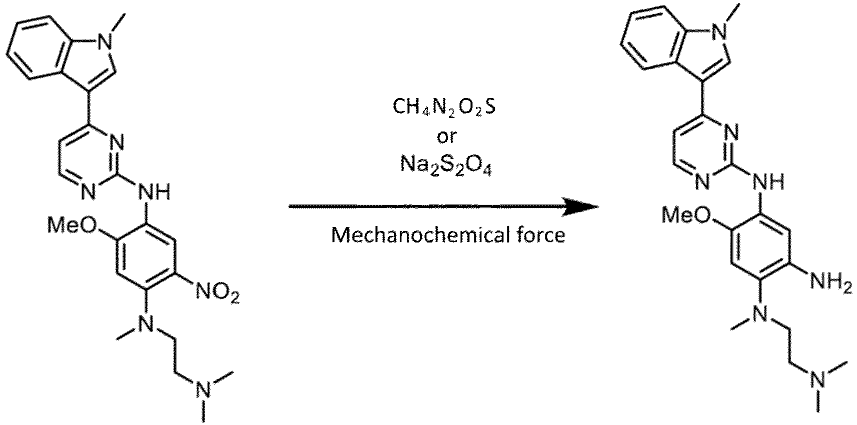 and,
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with acrylic acid, chloro-N,N,N',N'-tetramethylformamidinium hexafluorophosphate (TCFH), and tripotassium phosphate or potassium hydrogenophosphate, in liquid-assisted grinding (LAG) conditions, using BM to obtain a crude product containing osimertinib, followed by a purification of said crude product, to obtain osimertinib,

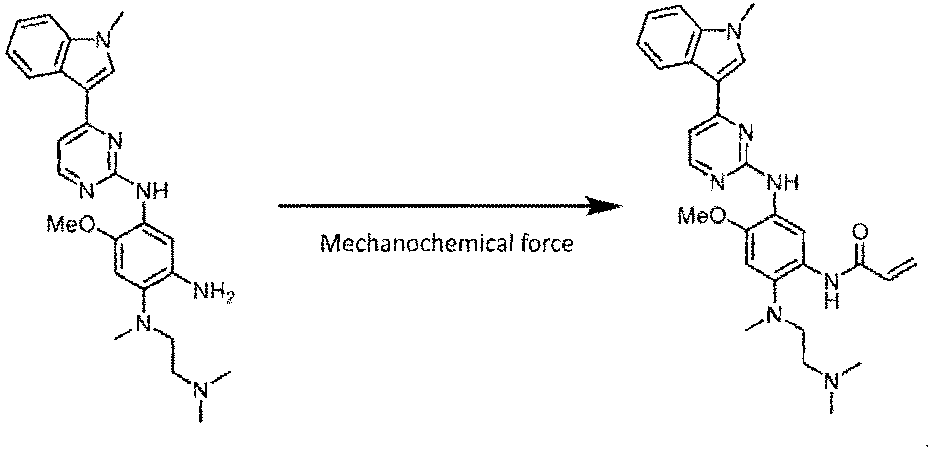

In this embodiment, the ball-milling is carried out in at least one device or a combination of devices chosen among: planetary ball-mill, drum mill, dyno-mill, or vibrating eccentric mill.

According to a particular embodiment, the process of the invention comprises the steps of:
a) a mechanochemical Friedel-Craft acylation step of 1-methylindole with 2,4-dichloropyrimidine and a Lewis acid, using RAM under neat grinding or in liquid-assisted grinding (LAG) conditions, to obtain a crude product containing 3-(2-chloropyrimidin-4-yl)-1-methyl-1H-indole, followed by a purification of said crude product, to obtain -(2-chloropyrimidin-4-yl)-1-methyl-1H-indole, 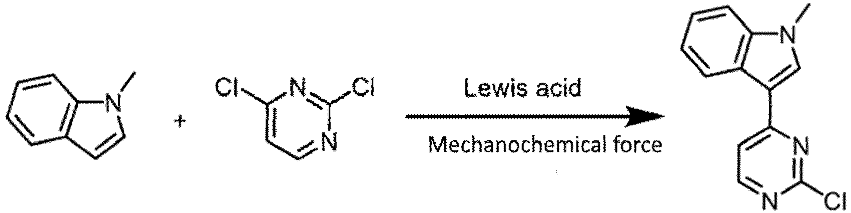
b) a mechanochemical nucleophilic aromatic substitution step of the above mentioned 3-(2-chloropyrimidin-4-yl)-1-methyl-1H-indole with 4-fluoro-2-methoxy-5-nitroaniline and a sulfonic acid, under neat grinding or in liquid-assisted grinding (LAG) conditions, using RAM to obtain a crude product containing N-(4-fluoro-2-methoxy-5-nitrophenyl)-4-(1-methyl-1H-indol-3-yl)pyrimidin-2-amine, followed by a purification of said crude product, to obtain N-(4-fluoro-2-methoxy-5-nitrophenyl)-4-(1-methyl-1H-indol-3-yl)pyrimidin-2-amine, 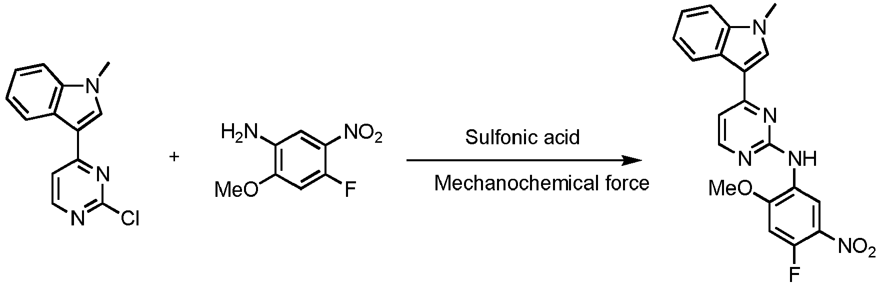
c) a mechanochemical nucleophilic aromatic substitution step of the above mentioned N-(4-fluoro-2-methoxy-5-nitrophenyl)-4-(1-methyl-1H-indol-3-yl)pyrimidin-2-amine with N,N,N'-trimethylethylenediamine and a base, in particular K₂CO₃, in liquid-assisted grinding (LAG) conditions, using RAM, to obtain a crude product containing N'-(2-dimethylaminoethyl)-2-methoxy-N'-methyl-N-[4-(1-methylindol-3-yl)pyrimidin-2-yl]-5-nitrobenzene-1,4-diamine, followed by a purification of said crude product, to obtain N'-(2-dimethylaminoethyl)-2-methoxy-N'-methyl-N-[4-(1-methylindol-3-yl)pyrimidin-2-yl]-5-nitrobenzene-1,4-diamine,

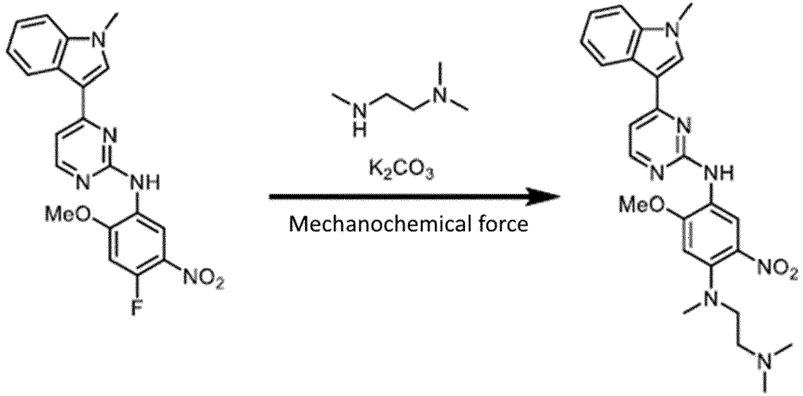

It can be noted that the reaction by BM and/or by RAM occurs at room temperature, while the reaction in solution (manufacturing route) occurs at 85°C. Our procedure is milder (no heating) than the manufacturing one in solution.
d) a mechanochemical reduction step of the above mentioned N'-(2-dimethylaminoethyl)-2-methoxy-N'-methyl-N-[4-(1-methylindol-3 -yl)pyrimidin-2-yl]-5-nitrobenzene-1,4-diamine with sodium dithionite or thiourea dioxide, under neat grinding or in liquid-assisted grinding (LAG) conditions, using RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 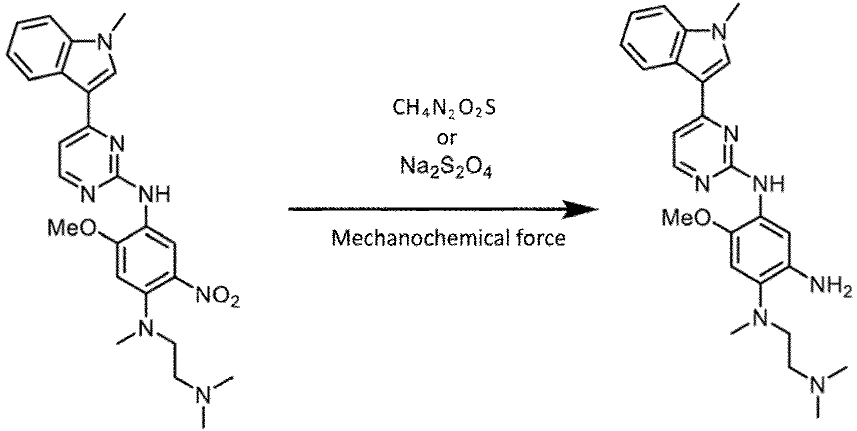 and,
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with potassium acrylate and (3-dimethylamino-propyl)-ethyl-carbodiimide hydrochloride, in liquid-assisted grinding (LAG) conditions, using RAM to obtain a crude product containing osimertinib, followed by a purification of said crude product, to obtain osimertinib,

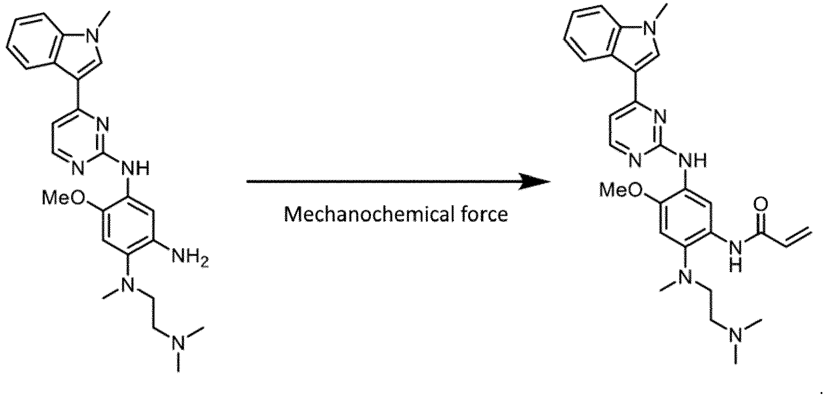

According to a preferred embodiment, in the process of the invention, in the step a), the Lewis acid used is AlCl₃,
and/or,
in the step b), the sulfonic acid used is camphorsulfonic acid.

According to a particular embodiment, in the process of the invention, in the step a), the Lewis acid used is AlCl₃.

According to a particular embodiment, in the process of the invention, in the step b), the sulfonic acid used is camphorsulfonic acid.

According to a preferred embodiment, in the process of the invention, in the step c), a polar solvent is added, preferably dimethylsulfoxide (DMSO),
and/or
in the step d), a mixture of ethanol and water is added,
   and/or
in the step e), a solvent chosen among ethyl acetate, 2-methyltetrahydrofuran, dimethyl sulfoxide, ethanol, glycerol, methyl-tert-butyl ether (MTBE), is added, preferably ethyl acetate.

According to a particular embodiment, in the process of the invention, in the step c), a polar solvent is added, preferably dimethylsulfoxide (DMSO).

According to a particular embodiment, in the process of the invention, the polar solvent is polyethylene glycol (PEG).

According to a particular embodiment, in the process of the invention, in the step d), a mixture of ethanol and water is added.

According to a particular embodiment, in the process of the invention, the volumetric ratio of the mixture of ethanol and water is from 5:1 to 1:1, preferably is 3:1.

According to a particular embodiment, in the process of the invention, in the step e), a solvent chosen among ethyl acetate, 2-methyltetrahydrofuran, dimethyl sulfoxide, ethanol, glycerol, methyl-tert-butyl ether (MTBE), is added, preferably ethyl acetate.

According to a preferred embodiment, in the process of the invention, in the step d), the N1-(2-dimethylaminoethyl)-5-methoxy-N1-methyl-N4-[4-(1-methylindol-3-yl)pyrimidin-2-yl]benzene-1,2,4-triamine is recovered from the crude product containing it, by a washing with ethanol, a filtration and an evaporation of said ethanol,
or,
in the step d), the N1-(2-dimethylaminoethyl)-5-methoxy-N1-methyl-N4-[4-(1-methylindol-3-yl)pyrimidin-2-yl]benzene-1,2,4-triamine is recovered from the crude product containing it, by an adjustment of the pH to 10 by using a 10% solution of K₂CO₃ (w/w), an extraction using ethyl acetate and an evaporation of said ethyl acetate.

According to a particular embodiment, in the process of the invention, in the step d), the N1-(2-dimethylaminoethyl)-5-methoxy-N1-methyl-N4-[4-(1-methylindol-3-yl)pyrimidin-2-yl]benzene-1,2,4-triamine is recovered from the crude product containing it, by a washing with ethanol, a filtration and an evaporation of said ethanol.

This embodiment is preferred when the step d) resorts to BM.

According to a particular embodiment, in the process of the invention, the N1-(2-dimethylaminoethyl)-5-methoxy-N1-methyl-N4-[4-(1-methylindol-3-yl)pyrimidin-2-yl]benzene-1,2,4-triamine is recovered from the crude product containing it, by an adjustment of the pH to 10 by using a 10% solution of K₂CO₃ (w/w), an extraction using ethyl acetate and an evaporation of said ethyl acetate.

This embodiment is preferred when the step d) resorts to RAM.

According to a preferred embodiment, in the process of the invention, the osimertinib is recovered from the crude product containing it, by a precipitation in water.

This embodiment concerns the step e) when resorts to BM or RAM.

This work-up also concerns step d (reduction) by both ball-mill and by RAM and this work-up is better than that one used in the manufacturing route for step d (reduction), where a purification by column chromatography is required.

According to a preferred embodiment, in the process of the invention, in step a), the mechanical forces are generated under neat grinding,
and/or
in step b), the mechanical forces are generated under neat grinding.

According to a particular embodiment, in the process of the invention, in step a), the mechanical forces are generated under neat grinding.

According to a particular embodiment, in the process of the invention, in step b), the mechanical forces are generated under neat grinding.

According to a preferred embodiment, in the process of the invention, in the step c), the LAG conditions are defined by the parameter η that is greater than 0 µL/mg and less than or equal to 1 µL/mg, more preferably is comprised from about 0.1 µL/mg to 0.5 µL/mg, more preferably 0.5 µL/mg,
and/or
in the step d), the LAG conditions are defined by the parameter η that is greater than 0 µL/mg and less than or equal to 1 µL/mg, more preferably is comprised from about 0.1 µL/mg to 0.5 µL/mg, more preferably 0.5 µL/mg,
   and/or,
in the step d), the LAG conditions are defined by the parameter η that is greater than 0 µL/mg and less than or equal to 1 µL/mg, more preferably is comprised from about 0.5 µL/mg to 1 µL/mg, more preferably 0.75 µL/mg,
   and/or
in the step e), the LAG conditions are defined by the parameter η that is greater than 0 µL/mg and less or equal to 1 µL/mg, more preferably is comprised from about 0.1 µL/mg to 0.5 µL/mg, more preferably 0.4 µL/mg, even more preferably 0.2 µL/mg.

According to a particular embodiment, in the process of the invention, in the step c), the LAG conditions are defined by the parameter η that is greater than 0 µL/mg and less than or equal to 1 µL/mg, more preferably is comprised from about 0.1 µL/mg to 0.5 µL/mg, more preferably 0.5 µL/mg.

According to a particular embodiment, in the process of the invention, in the step d), the LAG conditions are defined by the parameter η that is greater than 0 µL/mg and less than or equal to 1 µL/mg, more preferably is comprised from about 0.1 µL/mg to 0.5 µL/mg, more preferably 0.5 µL/mg.

According to a particular embodiment, in the process of the invention, in the step d), the LAG conditions are defined by the parameter η that is greater than 0 µL/mg and less than or equal to 1 µL/mg, more preferably is comprised from about 0.5 µL/mg to 1, more preferably 0.75 µL/mg. According to a particular embodiment, in the process of the invention, in the step e), the LAG conditions are defined by the parameter η that is greater than 0 µL/mg and less or equal to 1 µL/mg, more preferably is comprised from about 0.1 µL/mg to 0.5 µL/mg, more preferably 0.4 µL/mg, even more preferably 0.2 µL/mg.

According to a particular embodiment of the process of the invention, the LAG conditions are defined by the parameter η that is greater than 0 µL/mg and less than or equal to 1 µL/mg, more preferably is comprised from about 0.5 µL/mg to 1 µL/mg, more preferably 0.75 µL/mg, and in which the volumetric ratio of the mixture of ethanol and water is from 5:1 to 1:1, preferably is 3:1.

According to a preferred embodiment, in the process of the invention, comprises the step of: f) a salification step of the above mentioned osimertinib, in particular a mechanochemical salification with methanesulfonic acid using BM, to obtain osimertinib mesylate 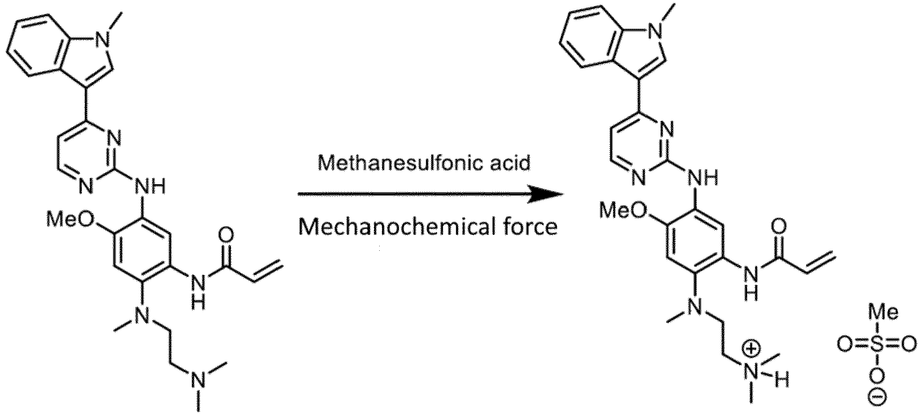 in particular in the step f), the mechanical forces are generated under neat grinding.

It can be noted that there is no work-up by ball-milling for this step (alternatively, a filtration by 2-Me-THF is conducted), while for the commercial route in solution, the work-up consists in a filtration by acetone. If the two work-up methods are compared for BM vs solution based methods, one can say the following:
a) no work-up needed (when the synthesis is done by ball-milling) is better than filtration in acetone, which is used when the synthesis is done in solution.
b) The filtration by 2-MeTHF (used after BM synthesis), is better that filtration is acetone, (used when the synthesis is done in solution). Indeed, 2-Me-THF is a biomass derivative solvent, which is a green alternative to ether solvents (e.g. THF, diethyl ether) and other organic volatile solvents. Therefore, 2.-Me-THF is in any case preferable to acetone (which is not green and more flammable and volatile than 2-Me-THF).

As a matter of example and in a non-limiting manner, the step f) can be carried out at 25°C.

According to a particular embodiment, the process of the invention comprises the steps of:
a) a mechanochemical Friedel-Craft acylation step of 1-methylindole with 2,4-dichloropyrimidine and a Lewis acid, using BM or RAM under neat grinding or in liquid-assisted grinding (LAG) conditions, to obtain a crude product containing 3-(2-chloropyrimidin-4-yl)-1-methyl-1H-indole, followed by a purification of said crude product, to obtain -(2-chloropyrimidin-4-yl)-1-methyl-1H-indole, 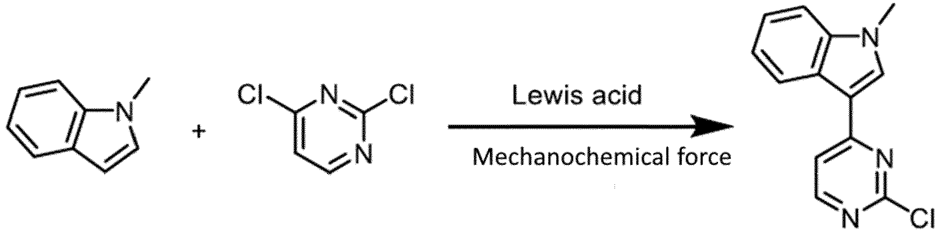
b)a mechanochemical nucleophilic aromatic substitution step of the above mentioned 3-(2-chloropyrimidin-4-yl)-1-methyl-1H-indole with 4-fluoro-2-methoxy-5-nitroaniline and a sulfonic acid, under neat grinding or in liquid-assisted grinding (LAG) conditions, using BM or RAM to obtain a crude product containing N-(4-fluoro-2-methoxy-5-nitrophenyl)-4-(1-methyl-1H-indol-3-yl)pyrimidin-2-amine, followed by a purification of said crude product, to obtain N-(4-fluoro-2-methoxy-5-nitrophenyl)-4-(1-methyl-1H-indol-3-yl)pyrimidin-2-amine, 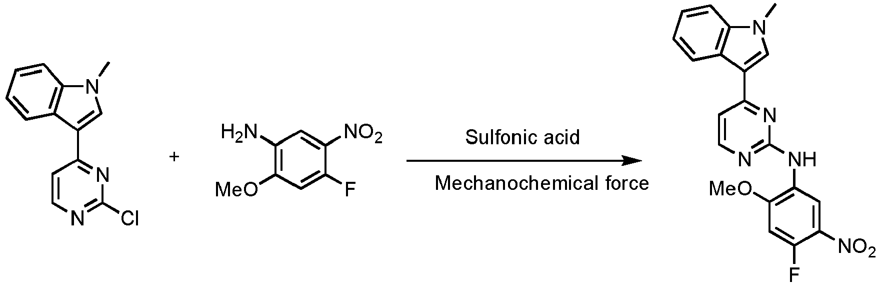
c) a mechanochemical nucleophilic aromatic substitution step of the above mentioned N-(4-fluoro-2-methoxy-5-nitrophenyl)-4-(1-methyl-1H-indol-3-yl)pyrimidin-2-amine with N,N,N'-trimethylethylenediamine and a base, in particular K₂CO₃, in liquid-assisted grinding (LAG) conditions, using BM or RAM, to obtain a crude product containing N'-(2-dimethylaminoethyl)-2-methoxy-N'-methyl-N-[4-(1-methylindol-3-yl)pyrimidin-2-yl]-5-nitrobenzene-1,4-diamine, followed by a purification of said crude product, to obtain N'-(2-dimethylaminoethyl)-2-methoxy-N'-methyl-N-[4-(1-methylindol-3-yl)pyrimidin-2-yl]-5-nitrobenzene-1,4-diamine,

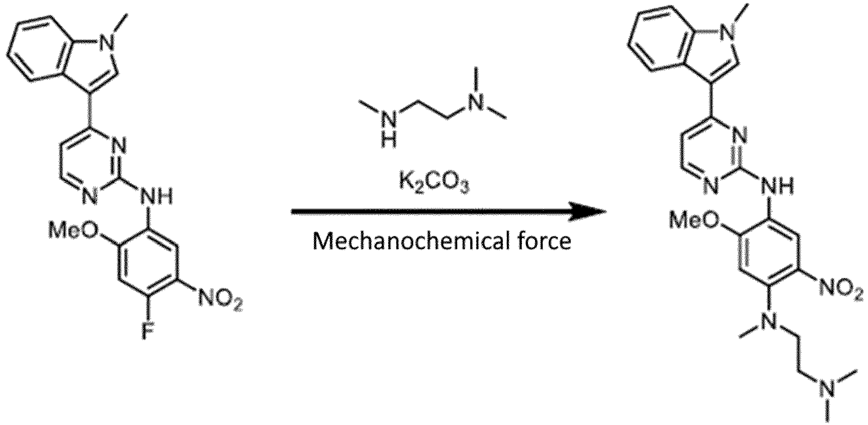

It can be noted that the reaction by BM and/or by RAM occurs at room temperature, while the reaction in solution (manufacturing route) occurs at 85°C. The present procedure is milder (no heating) than the manufacturing in solution.
d) a mechanochemical reduction step of the above mentioned N'-(2-dimethylaminoethyl)-2-methoxy-N'-methyl-N-[4-(1-methylindol-3-yl)pyrimidin-2-yl]-5-nitrobenzene-1,4-diamine with sodium dithionite or thiourea dioxide, under neat grinding or in liquid-assisted grinding (LAG) conditions, using BM or RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 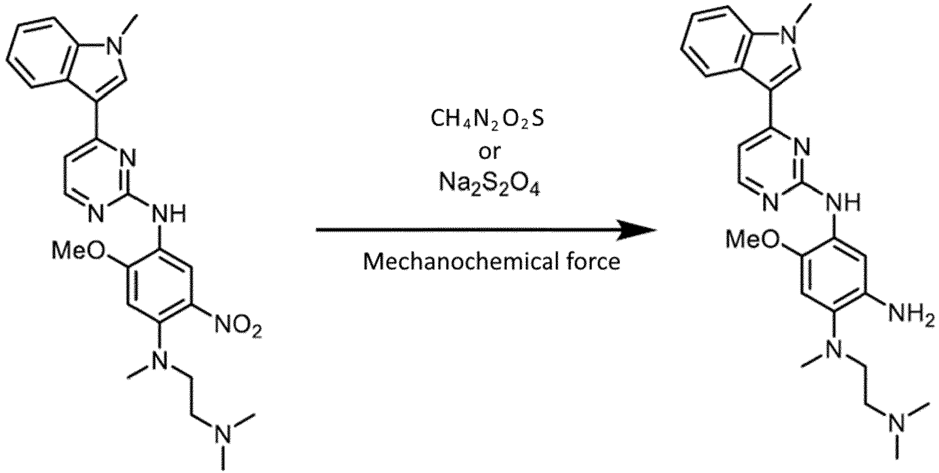
   e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with acrylic acid, chloro-N,N,N',N'-tetramethylformamidinium hexafluorophosphate (TCFH), and tripotassium phosphate or potassium hydrogenophosphate, in liquid-assisted grinding (LAG) conditions, using BM to obtain a crude product containing osimertinib, followed by a purification of said crude product, to obtain osimertinib,
      or
   e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with potassium acrylate and (3-dimethylamino-propyl)-ethyl-carbodiimide hydrochloride, in liquid-assisted grinding (LAG) conditions, using RAM to obtain a crude product containing osimertinib, followed by a purification of said crude product, to obtain osimertinib,
      and,
f) a salification step of the above mentioned osimertinib, in particular a mechanochemical salification with methanesulfonic acid using BM, to obtain osimertinib mesylate

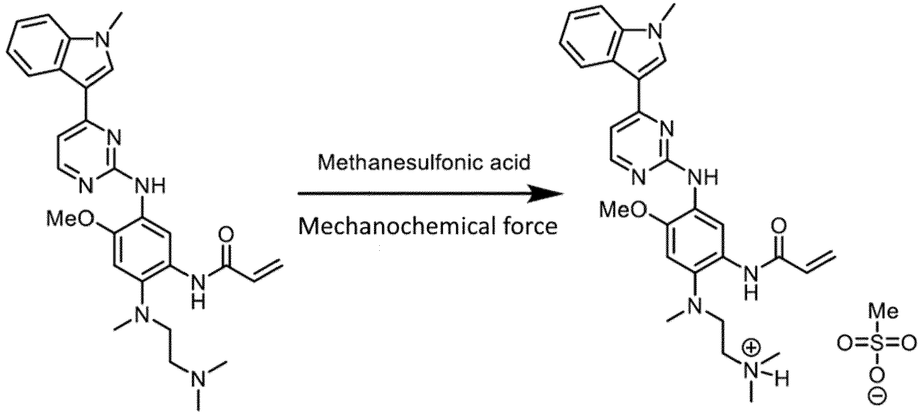

in particular in the step f), the mechanical forces are generated under neat grinding.

It can be noted that the mesylation reaction by BM occurs in 15 min, while the manufacturing route in solution required 90 min reaction.

According to a preferred embodiment, the process of the invention comprises the step of: f) a salification step of the above mentioned osimertinib, in particular a mechanochemical salification with methanesulfonic acid using RAM, to obtain a crude product containing osimertinib mesylate, followed by a purification of said crude product, to obtain osimertinib mesylate

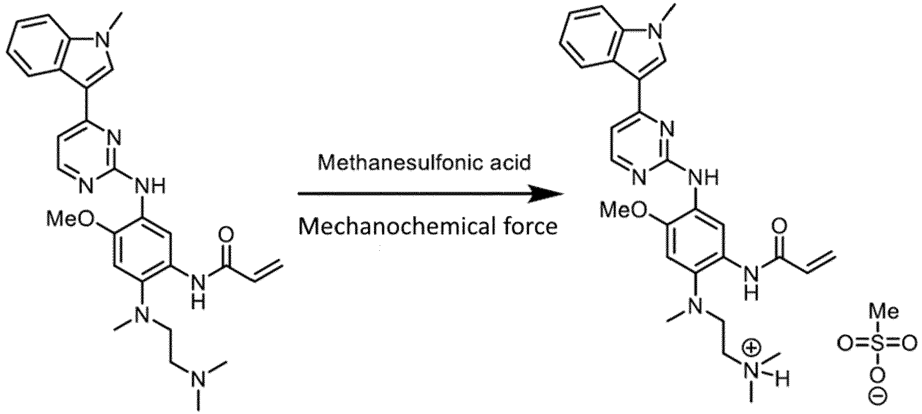

As a matter of example and in a non-limiting manner, the step f) can be carried out at 25°C with a mixture of isopropanol and water in LAG conditions.

It can be noted that the mesylation reaction by RAM occurs in 5 min, while the manufacturing route in solution required 90 min reaction.

It can be noted that the work up is done by precipitation in water (as the commercial manufacturing route).

According to a particular embodiment, the process of the invention comprises the steps of:
a) a mechanochemical Friedel-Craft acylation step of 1-methylindole with 2,4-dichloropyrimidine and a Lewis acid, using BM or RAM under neat grinding or in liquid-assisted grinding (LAG) conditions, to obtain a crude product containing 3-(2-chloropyrimidin-4-yl)-1-methyl-1H-indole, followed by a purification of said crude product, to obtain -(2-chloropyrimidin-4-yl)-1-methyl-1H-indole, 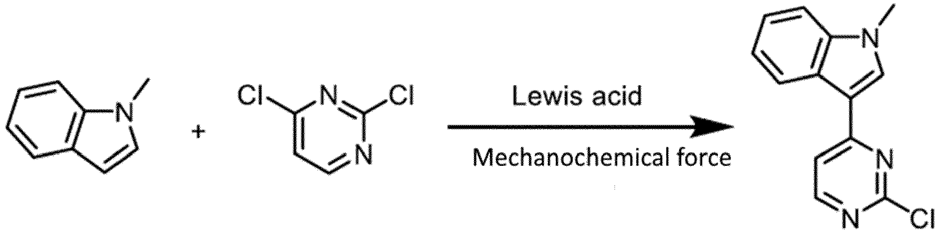
b) a mechanochemical nucleophilic aromatic substitution step of the above mentioned 3-(2-chloropyrimidin-4-yl)-1-methyl-1H-indole with 4-fluoro-2-methoxy-5-nitroaniline and a sulfonic acid, under neat grinding or in liquid-assisted grinding (LAG) conditions, using BM or RAM to obtain a crude product containing N-(4-fluoro-2-methoxy-5-nitrophenyl)-4-(1-methyl-1H-indol-3-yl)pyrimidin-2-amine, followed by a purification of said crude product, to obtain N-(4-fluoro-2-methoxy-5-nitrophenyl)-4-(1-methyl-1H-indol-3-yl)pyrimidin-2-amine, 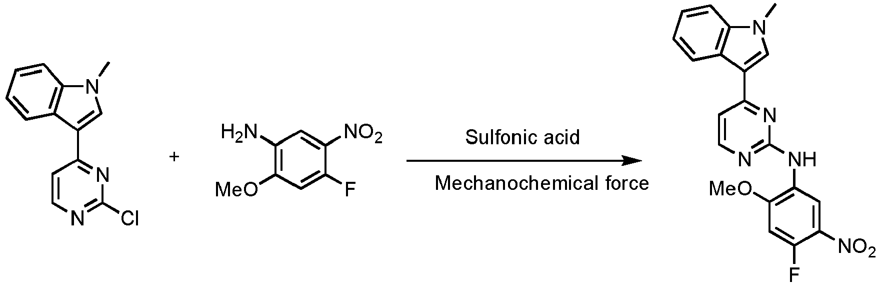
c) a mechanochemical nucleophilic aromatic substitution step of the above mentioned N-(4-fluoro-2-methoxy-5-nitrophenyl)-4-(1-methyl-1H-indol-3-yl)pyrimidin-2-amine with N,N,N'-trimethylethylenediamine and a base, in particular K₂CO₃, in liquid-assisted grinding (LAG) conditions, using BM or RAM, to obtain a crude product containing N'-(2-dimethylaminoethyl)-2-methoxy-N'-methyl-N-[4-(1-methylindol-3-yl)pyrimidin-2-yl]-5-nitrobenzene-1,4-diamine, followed by a purification of said crude product, to obtain N'-(2-dimethylaminoethyl)-2-methoxy-N'-methyl-N-[4-(1-methylindol-3-yl)pyrimidin-2-yl]-5-nitrobenzene-1,4-diamine,

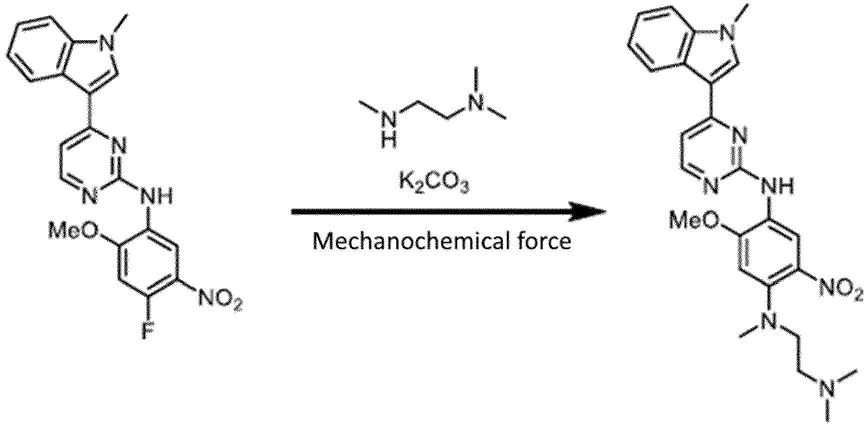

It can be noted thatthe reaction by BM and/or by RAM occurs at room temperature, while the reaction in solution (manufacturing route) occurs at 85°C. The present procedure is milder (no heating) than the manufacturing one in solution.
d) a mechanochemical reduction step of the above mentioned N'-(2-dimethylaminoethyl)-2-methoxy-N'-methyl-N-[4-(1-methylindol-3-yl)pyrimidin-2-yl]-5-nitrobenzene-1,4-diamine with sodium dithionite or thiourea dioxide, under neat grinding or in liquid-assisted grinding (LAG) conditions, using BM or RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 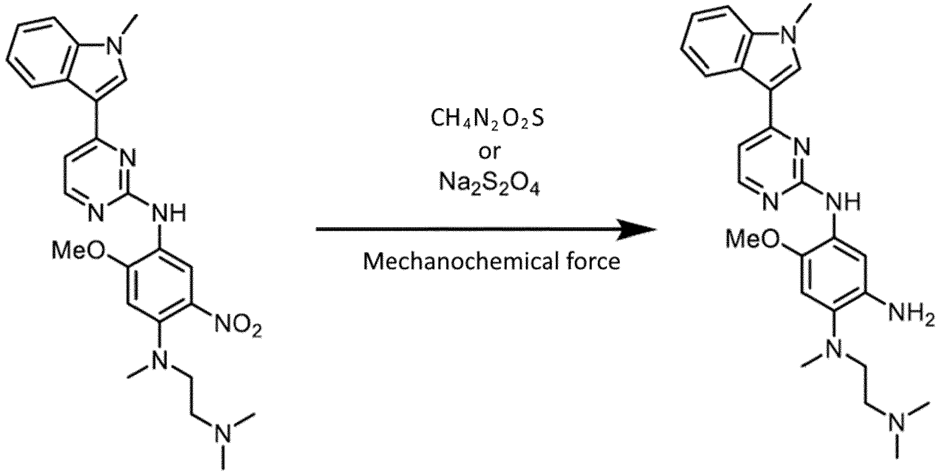
   e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with acrylic acid, chloro-N,N,N',N'-tetramethylformamidinium hexafluorophosphate (TCFH), and tripotassium phosphate or potassium hydrogenophosphate, in liquid-assisted grinding (LAG) conditions, using BM to obtain a crude product containing osimertinib, followed by a purification of said crude product, to obtain osimertinib,
      or
   e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with potassium acrylate and (3-dimethylamino-propyl)-ethyl-carbodiimide hydrochloride, in liquid-assisted grinding (LAG) conditions, using RAM to obtain a crude product containing osimertinib, followed by a purification of said crude product, to obtain osimertinib,
   and,
f) a salification step of the above mentioned osimertinib, in particular a mechanochemical salification with methanesulfonic acid using RAM, to obtain a crude product containing osimertinib mesylate, followed by a purification of said crude product, to obtain osimertinib mesylate

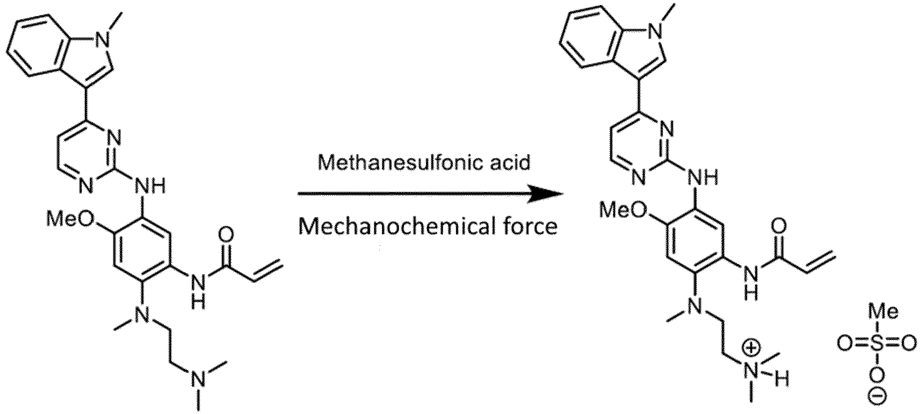

According to a preferred embodiment, in the process of the invention,
in the step f), 2-methyltetrahydrofuran is added
   and/or,
in the step f), the LAG conditions are defined by the parameter η that is greater than 0 µL/mg and less than or equal to 1 µL/mg, more preferably is comprised from about 0.1
µL/mg to 0.5 µL/mg, more preferably 0.4 µL/mg,
or,
in the step f), a mixture of an alcohol chosen among methanol, ethanol, isopropanol, polyol, glycerol or polyethylene glycol (PEGs), preferably isopropanol, and H₂O is added,
   and/or,
in the step f), the LAG conditions are defined by the parameter η that is greater than 0 µL/mg and less than or equal to 1 µL/mg, more preferably is comprised from about 0.1 µL/mg to 0.5 µL/mg, more preferably 0.3 µL/mg.

The expression "polyol" refers to compounds containing at least two alcohol functions that can be liquid under mechanochemical stress.

As a matter of example and in a non-limiting manner, PEG can be PEG-400, 600, 800, 1000, 2000, 3400, 5000, 20 000 etc. and also end-chain can be selected from HO-PEG-OH, MeO-PEG-OMe, or HO-PEG-OMe.

According to a particular embodiment, in the process of the invention, in the step f), 2-methyltetrahydrofuran is added.

According to a particular embodiment, in the process of the invention, in the step f), the LAG conditions are defined by the parameter η that is greater than 0 µL/mg and less than or equal to 1 µL/mg, more preferably is comprised from about 0.1 µL/mg to 0.5 µL/mg, more preferably 0.4 µL/mg.

According to a particular embodiment, in the process of the invention, in the step f), a mixture of an alcohol chosen among methanol, ethanol, isopropanol, polyol, glycerol or PEGs, preferably isopropanol, and H₂O is added.

According to a particular embodiment, in the process of the invention, in the step f), the LAG conditions are defined by the parameter η that is greater than 0 µL/mg and less than or equal to 1 µL/mg, more preferably is comprised from about 0.1 µL/mg to 0.5 µL/mg, more preferably 0.3 µL/mg.

According to a preferred embodiment, in the process of the invention, the osimertinib mesylate is recovered from the crude product containing it, by a filtration and a wash using a solvent, in particular acetone.

The present invention will be described in more detail with reference to examples below.

### Example 1 - Step d) by BM

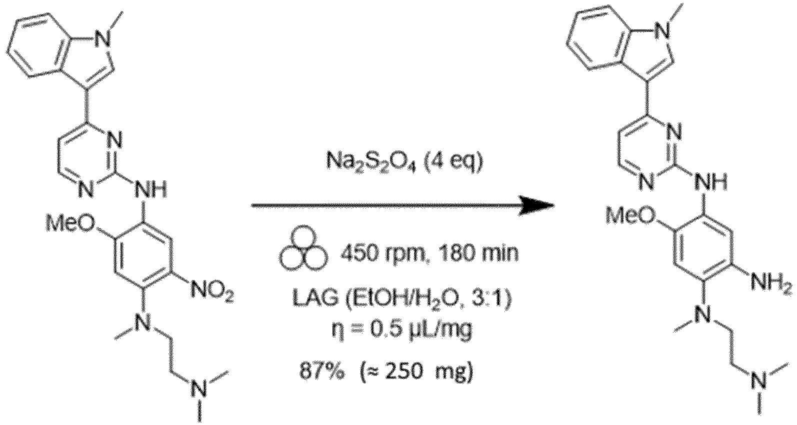

The starting material *N-*1-(2-(Dimethylamino)ethyl)-5-methoxy-*N*-1-methyl-*N-*4-(4-(1-methyl-1*H*-indol-3-yl)pyrimidin-2-yl)-2-nitrobenzene-1,4-diamine was obtained by mechanochemical synthesis but, it can be provided commercially.

*N*1-(2-(Dimethylamino)ethyl)-5-methoxy-*N-*1-methyl-*N*-4-(4-(1-methyl-1*H*-indol-3-yl)pyrimidin-2-yl)-2-nitrobenzene-1,4-diamine (300.0 mg, 0.63 mmol) and Na₂S₂O₄ (439.6 mg, 2.52 mmol, 4 eq) were placed in a 12 mL ZrO₂ jar charged with 25x5 mm ZrO₂ ball. Then, LAG additive was added (EtOH, 277 *µ*L; H₂O, 92 *µ*L, *η* = 0.5 µL/mg) and milled at 450 rpm for 180 min.

To the resulting reaction crude, EtOH (15 mL) was added and the mixture filtered. The solid residue was washed with additional EtOH (3x5 mL). The filtrate was collected, evaporated and dried to obtain N1-(2-dimethylaminoethyl)-5-methoxy-*N*-1-methyl-N4-[4-(1-methylindol-3-yl)pyrimidin-2-yl]benzene-1,2,4-triamine as an orange solid (245.1 mg, 87% yield).

¹H NMR (400 MHz, DMSO-*d₆*) δ (ppm): 9.88 (s, 1H), 8.92 (s, 1H), 8.51 (s, 1H), 8.43 (d, J = 8.0 Hz, 2H), 8.34-8.29 (m, 4H), 8.27 (d, J = 5 Hz, 2H), 8.16 (d, J = 8.0 Hz, 2H), 7.79 (s_{broad}, 2H), 7.75 (s_{broad}, 2H), 7.55-7.49 (d, J = 10.0 Hz, 6H), 7.28-7.13 (m, 13H), 6.89 (s, 2H), 6.79 (s, 2H), 3.92 (s, 6H), 3.88 (s, 6H), 3.82 (s, 6H), 3.75 (s, 7H), 3.14-3.03 (m, 12H), 2.88-2.77 (m, 9H), 2.61-2.57 (m, 12H).

### Example 2 - Step e) using TCFH and tripotassium phosphate by BM

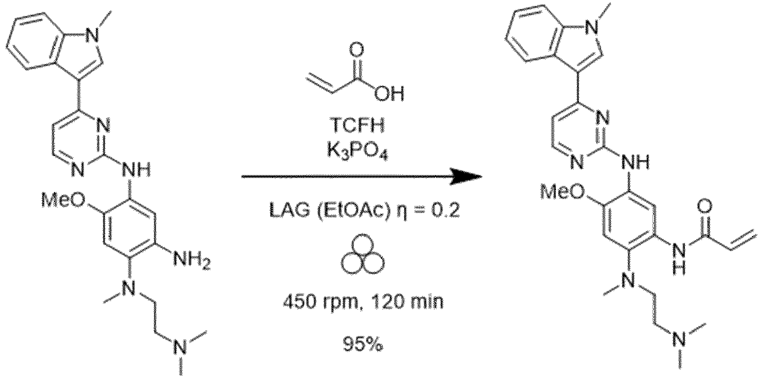

*N*1-(2-(Dimethylamino)ethyl)-5-methoxy-*N*1-methyl-*N-*4-(4-(1-methyl-1*H*-indol-3-yl)pyrimidin-2-yl)benzene-1,2,4-triamine (2000.2 mg, 4.49 mmol), acrylic acid (323 µL, 4.71 mmol, 1.05 eq), TCFH (1385.5 mg, 4.94 mmol, 1.1 eq) and K₃PO₄ (2858.8 mg, 13.47 mmol, 3 eq) were placed in a 45 mL ZrO₂ jar charged with 100x5 mm ZrO₂ balls. Then, LAG additive was added (EtOAc, 1316 µL, η = 0.2 µL/mg) and milled at 450 rpm for 120 min.

To the resulting reaction crude, water (50 mL) was added and the precipitate was isolated by filtration. The solid was washed with water (5x30 mL) and dried *in vacuo* in the presence of P₂O₅, to give osimertinib as a brown solid (2122.6 mg, 94% yield).

¹H NMR (400 MHz, DMSO-*d₆*) δ (ppm): 10.21 (s, 1 H), 9.16 (s, 1H), 8.69 (s, 1H), 8.34 (d, J = 5.3 Hz, 1H), 8.26 (d, J = 8.2 Hz, 1H), 7.92 (s, 1H), 7.54 (d, J = 8.2 Hz, 1H), 7.28-7.23 (m, 2H), 7.21-7.13 (m, 2H), 7.05 (s, 1H), 6.46 (dd, ³Jₜᵣₐₙₛ = 16.7 Hz, ³J_{cis} = 9.9 Hz, 1H), 6.29 (dd, ³Jₜᵣₐₙₛ = 16.7 Hz, ²J_{gem} = 2.0 Hz, 1 H), 5.79 (dd, ³J_{cis} = 9.9 Hz, ²J_{gem} = 2.0 Hz, 1 H), 3.93 (s, 3H), 3.87 (s, 3H), 2.96-2.88 (m, 2H), 2.72 (s, 3H), 2.37-2.32 (m, 2H), 2.24 (s, 6H).

### Example 3 - Step e) using TCFH and potassium hydrogenophosphate by BM

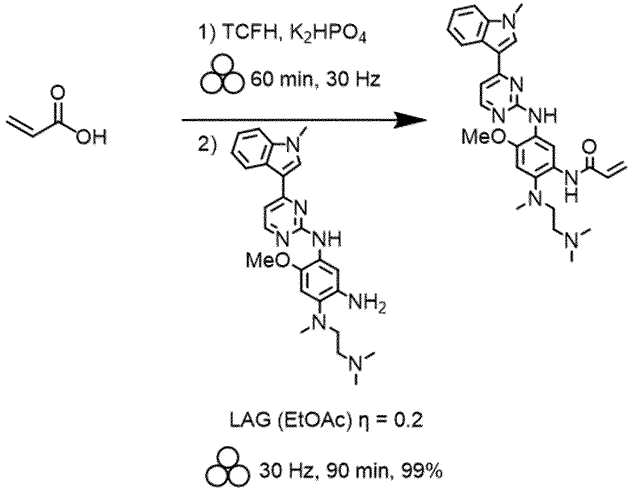

Acrylic acid (72 µL, 1.05 mmol, 1.05 eq), TCFH (308.6 mg, 1.1 mmol, 1.1 eq) and K₂HPO₄ (522.6 mg, 3.0 mmol, 3 eq) were placed in a 10 mL ZrO₂ jar charged with 1x10 mm ZrO₂ ball and milled at 30 Hz for 60 min. Then, *N-*1-(2-(Dimethylamino)ethyl)-5-methoxy-*N-*1-methyl-*N*4-(4-(1-methyl-1*H*-indol-3-yl)pyrimidin-2-yl)benzene-1,2,4-triamine (2000.2 mg, 4.49 mmol) and LAG additive (EtOAc, 270 µL, η = 0.2 µL/mg) were added and milled at 30 Hz for 90 min. To the resulting reaction crude, water (30 mL) was added and the precipitate was isolated by filtration. The solid was washed with water (6x20 mL) and dried *in vacuo* in the presence of P₂O₅, to give osimertinib as a brown solid (493.2 mg, 99% yield).

The NMR data are the same than Example 2.

### Example 4 - Step f) under neat grinding by BM

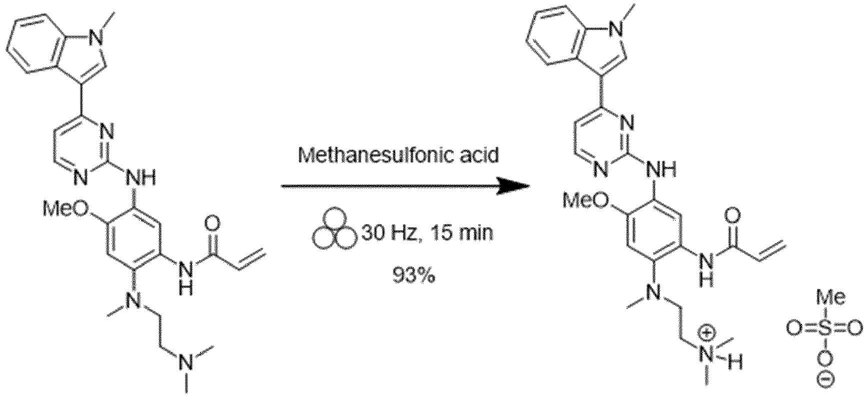

Osimertinib (350.0 mg, 0.70 mmol) and methanesulfonic acid (45 µL, 0.70 mmol, 1.0 eq) were placed in a 10 mL Teflon^{™} jar charged with 1x10 mm ZrO₂ ball and milled at 30 Hz for 15 min. The resulting solid was recovered without further workup to give osimertinib mesylate as a brown solid (387.7 mg, 93% yield).

Optionally, if a sticky solid was obtained as the reaction crude (due to the presence of humidity in the atmosphere and in the starting material), 15 mL 2-methyltetrahydrofuran (2-MeTHF) were added and the precipitate was recovered by filtration. The solid was washed with 2-MeTHF (5x5 mL) and dried in the air to yield osimertinib mesylate.

¹H NMR (400 MHz, DMSO-*d₆*) δ (ppm): 9.50 (s, 1 H), 9.30 (s_{broad}, 1H), 8.76 (s, 1H), 8.34 (s_{broad}, 2H), 7.58 (d, J = 8.5 Hz, 1H), 7.39 (d, J = 6.4 Hz, 1H), 7.34-7.25 (m, 2H), 7.22-7.13 (m, 2H), 7.08 (s, 1H), 6.74 (dd, ³Jₜᵣₐₙₛ = 16.8 Hz, ³J_{cis} = 10.5 Hz, 1H), 6.29 (d, ³Jₜᵣₐₙₛ = 16.7 Hz, 1 H), 5.79 (d, ³J_{cis} = 10.5 Hz, 1 H), 3.92 (s, 3H), 3.86 (s, 3H), 2.98-2.92 (s, 2H), 2.85 (s, 6H), 2.707-2.62 (m, 2H), 2.39 (s, 6H).

### Example 5 - Step d) by RAM

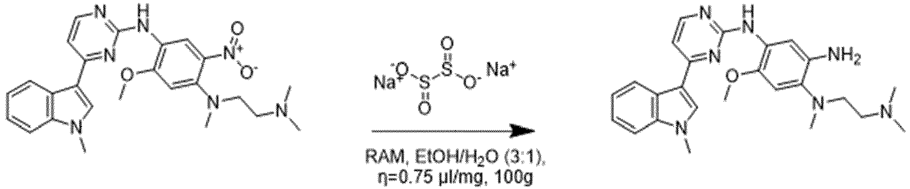

The starting material *N*1-(2-(Dimethylamino)ethyl)-5-methoxy-*N*1-methyl-N4-(4-(1-methyl-1*H*-indol-3-yl)pyrimidin-2-yl)-2-nitrobenzene-1,4-diamine was obtained by mechanochemical synthesis but, it can be provided commercially.

700mg (1.47mmol, 1eq) of the *N*1-(2-(Dimethylamino)ethyl)-5-methoxy-*N*1-methyl-N4-(4-(1-methyl-1*H*-indol-3-yl)pyrimidin-2-yl)-2-nitrobenzene-1,4-diamine and 1.03 g of sodium dithionite (5.89 mmol, 4 eq) were added in a 5 mL PTFE jar. Then EtOH (970 µL) and water (324 µL) were added in a ratio of 3:1 and of a total η value of 0.75 µL/mg. Then the vessel was stirred in a LabRAM II Resodyn mixer of the company Resodyn^{™} at 100 g for 30 minutes. The crude reaction mixture was dissolved in water and the pH was adjusted at 10 with a 10% (w/w) solution of K₂CO₃. Then the dissolved crude reaction mixture was extracted with EtOAc and the organic phase was washed with brine, isolated and dried over MgSO₄. Then it was evaporated under reduced pressure to give N1-(2-dimethylaminoethyl)-5-methoxy-N1-methyl-N4-[4-(1-methylindol-3-yl)pyrimidin-2-yl]benzene-1,2,4-triamine as a grey powder (368.9mg, 55%).

¹H NMR (400 MHz, DMSO-*d₆*) δ (ppm) : 8.42 (d, *J* = 7.8 Hz, 1H), 8.30 (s, 1H), 8.27 (d, *J* = 5.4 Hz, 1H), 7.78 (s, 1H), 7.54 - 7.47 (m, 2H), 7.24 (t, *J* = 7.6 Hz, 1H), 7.16 (dd, *J* = 11.4, 6.4 Hz, 2H), 6.76 (s, 1H), 3.88 (s, 3H), 3.74 (s, 3H), 2.88 (t, *J* = 6.7 Hz, 2H), 2.63 (s, 3H), 2.35 (t, *J* = 6.7 Hz, 2H), 2.17 (s, 6H).

¹³C NMR (101 MHz, DMSO-*d₆*) δ (ppm) : 162.66, 160.98, 157.55, 142.38, 138.17, 137.27, 133.89, 133.38, 126.12, 125.73, 122.78, 122.62, 121.39, 112.97, 110.8, 109.57, 107.29, 105.77, 66.88, 57.89, 56.96, 54.53, 46.24, 42.19, 33.49.

HRMS ESI (+) *m*/*z:* Calcd. For C₂₅H₃₂N₇O [M+ H]⁺, 446.2668; found, 446.2663.

### Example 6 - Step e) using N-Ethyl-N'-(3-dimethylaminopropyl) carbodiimide hydrochloride by RAM

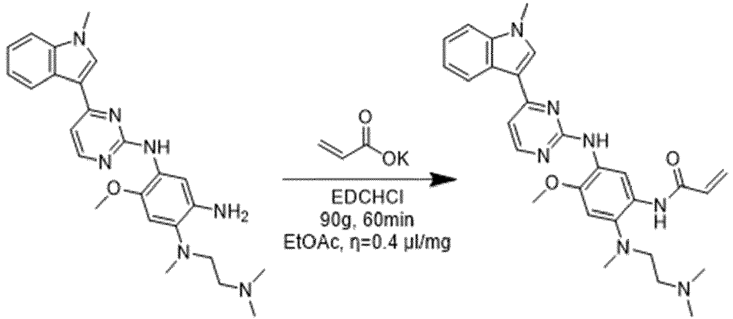

N1-(2-(Dimethylamino) ethyl) -5-methoxy-N1-methyl-N4-(4-(1-methyl-1H-indol-3-yl) pyrimidin-2-yl) benzene-1,2,4-triamine (1g, 2.24 mmol), potassium acrylate (271.9mg, 2.47 mmol, 1.1 eq) and N-Ethyl-N'-(3-dimethylaminopropyl) carbodiimide hydrochloride (EDCHCl) (516.3 mg, 2.69 mmol, 1.2 eq) and EtOAc (715 µl, η=0.4 µL/mg) were added in a 10 mL glass vial and the vial was stirred in a LabRAM II Resodyn mixer of the company Resodyn^{™} at 90 g for 60 minutes. After the end of the mixing, the resulting crude was transferred to a 100 mL beaker and distilled water (50 mL) was added and a brown precipitate was formed. Then it was filtered under vacuum and the resulting precipitate was washed further with 100 mL of distilled water, and the water was dried in vacuo in the presence of P₂O₅, to give osimertinib as a brown solid (1.08 g, 97% yield).

¹H NMR (400 MHz, DMSO-*d₆*) δ (ppm) 10.21 (s, 1 H), 9.16 (s, 1H), 8.69 (s, 1H), 8.34 (d, J = 5.3 Hz, 1H), 8.26 (d, J = 8.2 Hz, 1H), 7.92 (s, 1H), 7.54 (d, J = 8.2 Hz, 1H), 7.28-7.23 (m, 2H), 7.21-7.13 (m, 2H), 7.05 (s, 1H), 6.46 (dd, J = 16.7 Hz, J = 9.9 Hz, 1H), 6.29 (dd, J = 16.7 Hz, J = 2.0 Hz, 1 H), 5.79 (dd, J = 9.9 Hz, J= 2.0 Hz, 1 H), 3.93 (s, 3H), 3.87 (s, 3H), 2.96-2.88 (m, 2H), 2.72 (s, 3H), 2.37-2.32 (m, 2H), 2.24 (s, 6H).

### Example 7 - Step f) with i-PrOH/H₂O by RAM

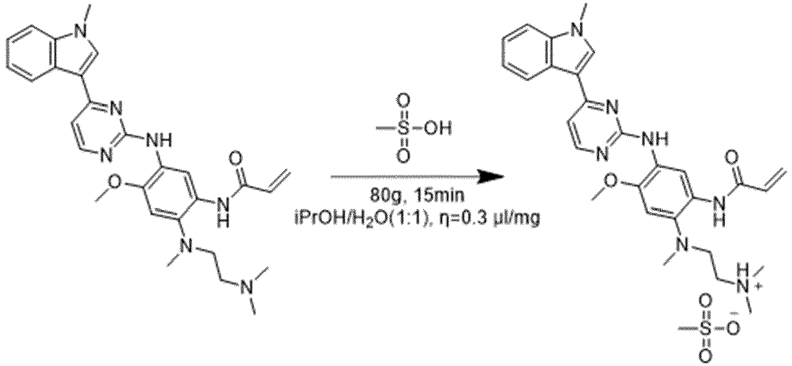

In a 4 mL polypropylene vial, osimertinib (338.1 mg, 0.68 mmol) and methanesulfonic acid (71.5 mg, 0.74 mmol, 48.3 *µ*l) and iPrOH/H₂O (1:1) (123 *µ*L, *η* = 0.3 *µ*L/mg) were added and mixed at 80 *g* for 5 minutes in a LabRAM II Resodyn mixer of the company Resodyn ^{™}. After the end of the mixing, the resulting crude was washed with 20 mL acetone and a yellow precipitate was formed. Then it was filtered under vacuum and the resulting precipitate was washed further with 30 mL of acetone and dried in vacuo to give osimertinib mesylate as a yellow solid (396.7 mg, 98% yield).

¹H NMR (400 MHz, DMSO-*d₆*) δ (ppm) 9.50 (s, 1 H), 9.30 ( 1H), 8.76 (s, 1H), 8.34 (2H), 7.58 (d, J = 8.5 Hz, 1H), 7.39 (d, J = 6.4 Hz, 1H), 7.34-7.25 (m, 2H), 7.22-7.13 (m, 2H), 7.08 (s, 1H), 6.74 (dd, J= 16.8 Hz, J = 10.5 Hz, 1H), 6.29 (d, J= 16.7 Hz, 1 H), 5.79 (d, J= 10.5 Hz, 1 H), 3.92 (s, 3H), 3.86 (s, 3H), 2.98-2.92 (s, 2H), 2.85 (s, 6H), 2.707-2.62 (m, 2H), 2.39 (s, 6H).

## Claims

1. Use of a mechanical force to generate a mechanochemical reaction in the implementation of a process of preparation, of *N-(2-*{[2(dimethylamino)ethyl](methyl)amino}-4-methoxy-5-{[4-(1-methyl-1*H*-indol-3-yl)pyrimidin-2-yl]amino}phenyl)prop-2-enamide (osimertinib) or a pharmaceutically acceptable salt thereof, comprising n steps, n being 5 or 6, from 1-methylindole, wherein at least one step is mechanochemical.

2. The use according to claim 1, wherein the mechanical forces are generated by a method chosen among: ball-milling (BM) or resonant acoustic mixing (RAM).

3. The use according to any one of claims 1 or 2, wherein at least two steps, three steps, four steps, five steps or all the steps are mechanochemical.

4. The use according to any one of claims 1 to 3, comprising a reduction step of N'-(2-dimethylaminoethyl)-2-methoxy-N'-methyl-N-[4-(1-methylindol-3 -yl)pyrimidin-2-yl]-5-nitrobenzene-1,4-diamine into N1-(2-dimethylaminoethyl)-5-methoxy-N1-methyl-N4-[4-(1-methylindol-3-yl)pyrimidin-2-yl]benzene-1,2,4-triamine, said reduction step is mechanochemical,
and/or
comprising an amidation step of the above mentioned N1-(2-dimethylaminoethyl)-5-methoxy-N1-methyl-N4-[4-(1-methylindol-3-yl)pyrimidin-2-yl]benzene-1,2,4-triamine into osimertinib, said amidation step is mechanochemical.

5. A mechanochemical process of preparation of osimertinib, which comprises the step of:
d) a mechanochemical reduction step of N'-(2-dimethylaminoethyl)-2-methoxy-N'-methyl-N-[4-(1-methylindol-3-yl)pyrimidin-2-yl]-5-nitrobenzene-1,4-diamine with sodium dithionite or thiourea dioxide, under neat grinding or in liquid-assisted grinding (LAG) conditions, using BM or RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine.

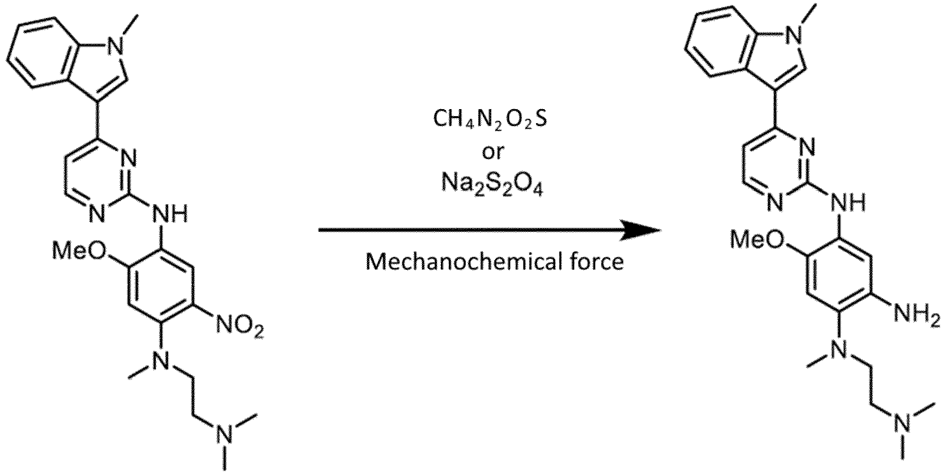

6. The mechanochemical process according to claim 5, comprises the steps of:
a) a mechanochemical Friedel-Craft acylation step of 1-methylindole with 2,4-dichloropyrimidine and a Lewis acid, using BM or RAM under neat grinding or in liquid-assisted grinding (LAG) conditions, to obtain a crude product containing 3-(2-chloropyrimidin-4-yl)-1-methyl-1H-indole, followed by a purification of said crude product, to obtain -(2-chloropyrimidin-4-yl)-1-methyl-1H-indole, 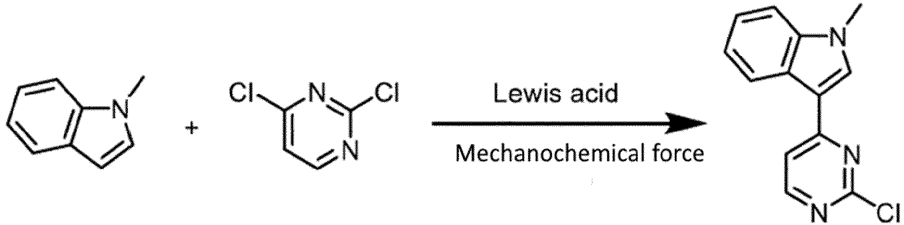
b) a mechanochemical nucleophilic aromatic substitution step of the above mentioned 3-(2-chloropyrimidin-4-yl)-1-methyl-1H-indole with 4-fluoro-2-methoxy-5-nitroaniline and a sulfonic acid, under neat grinding or in liquid-assisted grinding (LAG) conditions, using BM or RAM to obtain a crude product containing N-(4-fluoro-2-methoxy-5-nitrophenyl)-4-(1-methyl-1H-indol-3-yl)pyrimidin-2-amine, followed by a purification of said crude product, to obtain N-(4-fluoro-2-methoxy-5-nitrophenyl)-4-(1-methyl-1H-indol-3-yl)pyrimidin-2-amine, 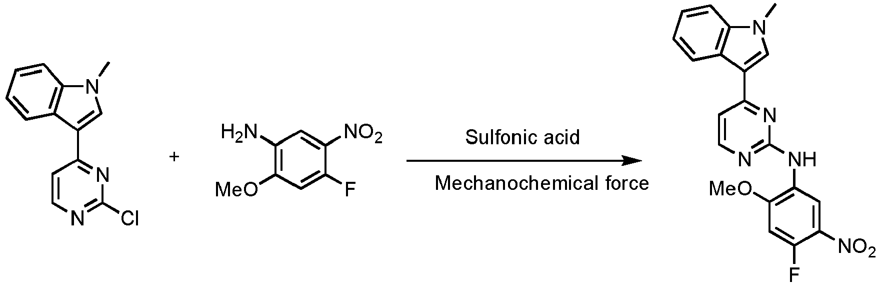
c) a mechanochemical nucleophilic aromatic substitution step of the above mentioned N-(4-fluoro-2-methoxy-5-nitrophenyl)-4-(1-methyl-1H-indol-3-yl)pyrimidin-2-amine with N,N,N'-trimethylethylenediamine and a base, in particular K₂CO₃, in liquid-assisted grinding (LAG) conditions, using BM or RAM, to obtain a crude product containing N'-(2-dimethylaminoethyl)-2-methoxy-N'-methyl-N-[4-(1-methylindol-3-yl)pyrimidin-2-yl]-5-nitrobenzene-1,4-diamine, followed by a purification of said crude product, to obtain N'-(2-dimethylaminoethyl)-2-methoxy-N'-methyl-N-[4-(1-methylindol-3-yl)pyrimidin-2-yl]-5-nitrobenzene-1,4-diamine, 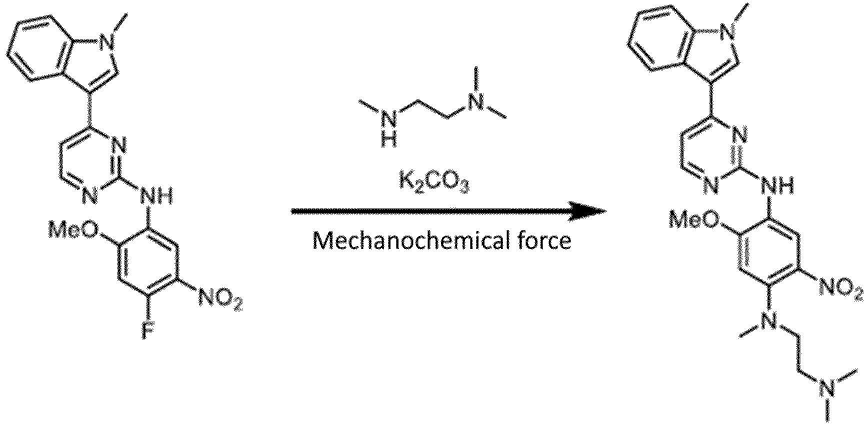
d) a mechanochemical reduction step of the above mentioned N'-(2-dimethylaminoethyl)-2-methoxy-N'-methyl-N-[4-(1-methylindol-3 -yl)pyrimidin-2-yl]-5-nitrobenzene-1,4-diamine with sodium dithionite or thiourea dioxide, under neat grinding or in liquid-assisted grinding (LAG) conditions, using BM or RAM, to obtain a crude product containing N1-(2-dimethylaminoethyl)-5-methoxy-N1-methyl-N4-[4-(1-methylindol-3-yl)pyrimidin-2-yl]benzene-1,2,4-triamine, followed by a purification of said crude product, to obtain N1-(2-dimethylaminoethyl)-5-methoxy-N1-methyl-N4-[4-(1-methylindol-3-yl)pyrimidin-2-yl]benzene-1,2,4-triamine, 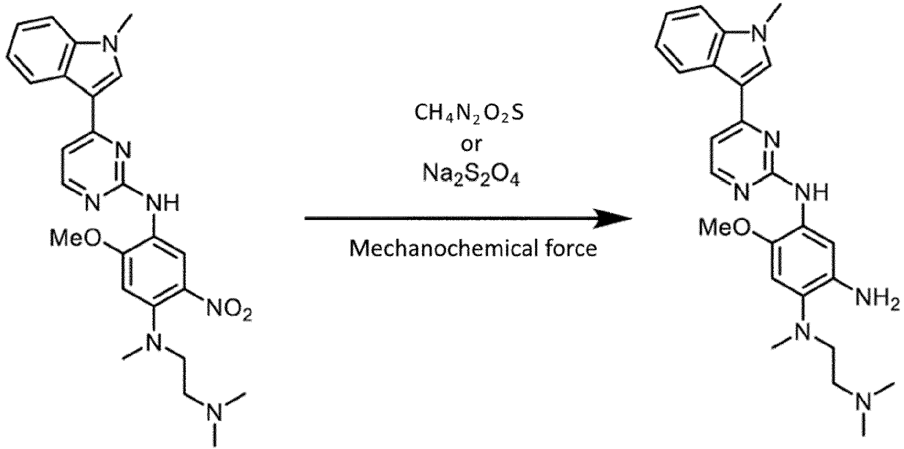 and,
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with acrylic acid, chloro-N,N,N',N'-tetramethylformamidinium hexafluorophosphate (TCFH), and tripotassium phosphate or potassium hydrogenophosphate, in liquid-assisted grinding (LAG) conditions, using BM to obtain a crude product containing osimertinib, followed by a purification of said crude product, to obtain osimertinib,
or
e) a mechanochemical amidation step of the above mentioned N1-(2-Dimethylaminoethyl)-5-methoxy-N1-methyl-N4-[4-(1-methylindol-3-yl)pyrimidin-2-yl]benzene-1,2,4-triamine with potassium acrylate and (3-dimethylamino-propyl)-ethyl-carbodiimide hydrochloride, in liquid-assisted grinding (LAG) conditions, using RAM to obtain a crude product containing osimertinib, followed by a purification of said crude product, to obtain osimertinib,

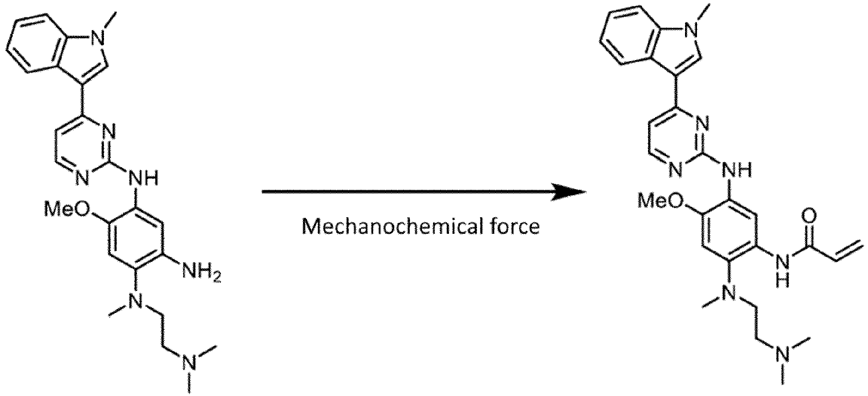

7. The mechanochemical process according to claim 6, wherein in the step a), the Lewis acid is AlCl₃,
and/or,
wherein in the step b), the sulfonic acid is camphorsulfonic acid.

8. The mechanochemical process according to any one of claims 6 to 7, wherein in the step c), a polar solvent is added, preferably dimethylsulfoxide (DMSO),
and/or
wherein in the step d), a mixture of ethanol and water is added,
and/or
wherein in the step e), a solvent chosen among ethyl acetate, 2-methyltetrahydrofuran, dimethyl sulfoxide, ethanol, glycerol, methyl-tert-butyl ether (MTBE), is added, preferably ethyl acetate.

9. The mechanochemical process according to any one of claims 6 to 8, wherein in the step d), wherein the N1-(2-dimethylaminoethyl)-5-methoxy-N1-methyl-N4-[4-(1-methylindol-3-yl)pyrimidin-2-yl]benzene-1,2,4-triamine is recovered from the crude product containing it, by a washing with ethanol, a filtration and an evaporation of said ethanol,
or,
wherein in the step d), wherein the N1-(2-dimethylaminoethyl)-5-methoxy-N1-methyl-N4-[4-(1-methylindol-3-yl)pyrimidin-2-yl]benzene-1,2,4-triamine is recovered from the crude product containing it, by an adjustment of the pH to 10 by using a 10% solution of K₂CO₃ (w/w), an extraction using ethyl acetate and an evaporation of said ethyl acetate.

10. The mechanochemical process according to any one of claims 6 to 9, wherein in step a), the mechanical forces are generated under neat grinding,
and/or
wherein in step b), the mechanical forces are generated under neat grinding.

11. The mechanochemical process according to any one of claims 6 to 10, wherein in the step c), the LAG conditions are defined by the parameter η that is greater than 0 µL/mg and less than or equal to 1 µL/mg, more preferably is comprised from about 0.1 µL/mg to µL/mg, more preferably 0.5 µL/mg,
and/or
wherein in the step d), the LAG conditions are defined by the parameter η that is greater than 0 µL/mg and less than or equal to 1 µL/mg, more preferably is comprised from about 0.1 µL/mg to 0.5 µL/mg, more preferably 0.5 µL/mg,
and/or,
wherein in the step d), the LAG conditions are defined by the parameter η that is greater than 0 µL/mg and less than or equal to 1 µL/mg, more preferably is comprised from about 0.5 µL/mg to 1 µL/mg, more preferably 0.75 µL/mg,
and/or
wherein in the step e), the LAG conditions are defined by the parameter η that is greater than 0 µL/mg and less or equal to 1 µL/mg, more preferably is comprised from about 0.1 µL/mg to 0.5 µL/mg, more preferably 0.4 µL/mg, even more preferably 0.2 µL/mg.

12. The mechanochemical process according to any one of claims 6 to 11, comprises the step of :
f) a salification step of the above mentioned osimertinib, in particular a mechanochemical salification with methanesulfonic acid using BM, to obtain osimertinib mesylate 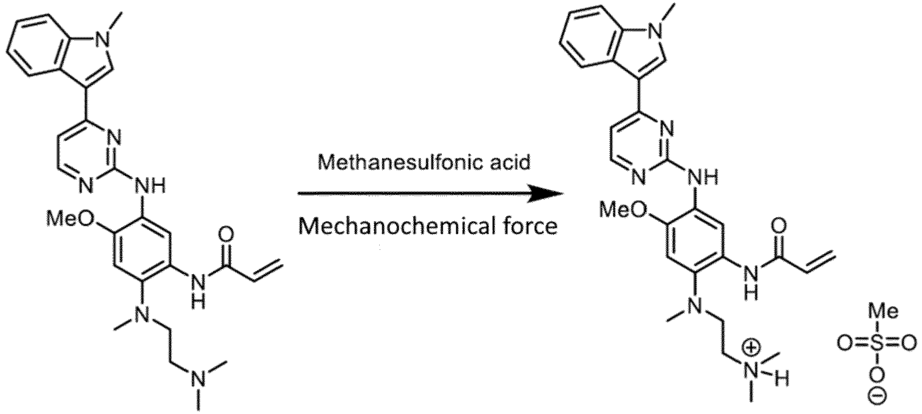 in particular in the step f), the mechanical forces are generated under neat grinding.

13. The mechanochemical process according to any one of claims 6 to 11, comprises the step of :
f) a salification step of the above mentioned osimertinib, in particular a mechanochemical salification with methanesulfonic acid using RAM, to obtain a crude product containing osimertinib mesylate, followed by a purification of said crude product, to obtain osimertinib mesylate

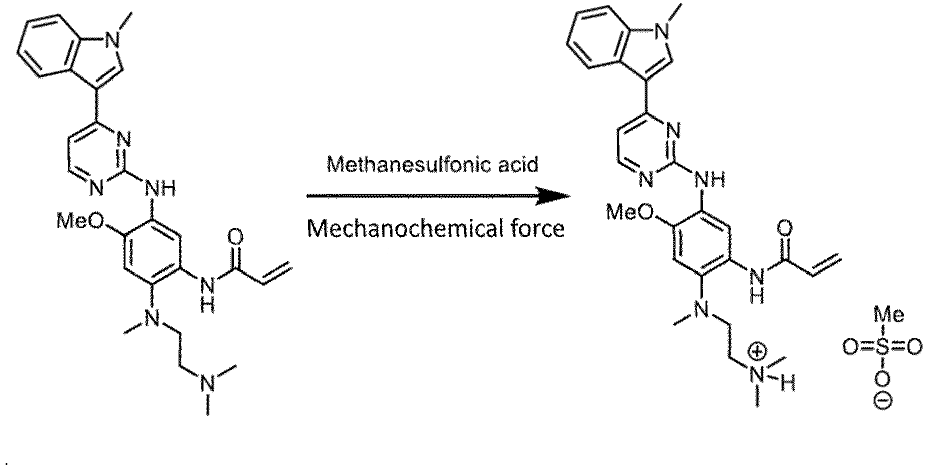

14. The mechanochemical process according to claim 11,
wherein in the step f), 2-methyltetrahydrofuran is added and/or,
wherein in the step f), the LAG conditions are defined by the parameter η that is greater than 0 µL/mg and less than or equal to 1 µL/mg, more preferably is comprised from about 0.1 µL/mg to 0.5 µL/mg, more preferably 0.4 µL/mg, or,
wherein in the step f), a mixture of an alcohol chosen among methanol, ethanol, isopropanol, polyol, glycerol or polyethylene glycol (PEGs), preferably isopropanol, and H₂O is added,
and/or,
wherein in the step f), the LAG conditions are defined by the parameter η that is greater than 0 µL/mg and less than or equal to 1 µL/mg, more preferably is comprised from about 0.1 µL/mg to 0.5 µL/mg, more preferably 0.3 µL/mg.

15. The mechanochemical process according to any one of claims 13 to 14, the osimertinib mesylate is recovered from the crude product containing it, by a filtration and a wash using a solvent, in particular acetone.
